# DEMANDE DE BREVET EUROPEEN

(11) **EP 0 376 819 A1**
(43) Date de publication de la demande: **04.07.1990**
(21) Numéro de dépôt: 89403614.4
(22) Date de dépôt: 22.12.1989
(51) Int. Cl.: C07C 235/10, C07C 279/12, C07C 279/24, C07D 209/20, C07D 311/14, A01N 37/20, A01N 43/16, A01N 43/38

(54) **Nouvelles polyamines acylées, leur procédé de préparation et leur application comme fongicides**

(30) Priorité: 22.12.1988 FR 8816994
(71) Demandeur: ROUSSEL-UCLAF, 75007 Paris (FR)
(72) Inventeur: Brayer, Jean-Louis, F-60440 Nanteuil Le Haudoin (FR); Taliani, Laurent, F-93320 Pavillons Sous Bois (FR); Tessier, Jean, F-94300 Vincennes (FR)
(74) Mandataire: Tonnellier, Marie-José

(57) **Abrégé**

L'invention concerne les composés de formule générale (I) : 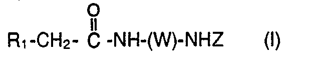 dans lesquels,
R, représente :
   soit un radical ArO- dans lequel Ar représente un aryle, hétéroaryle ou hétérocycle,

   - soit un radical aryle, polyaryle ou hétérocyclique.
W représente :
   - soit (CH₂)ₙ₁,
   - soit (CH₂)n₂ NY(CH₂)n₃, Y représentant un hydrogène ou un radical alkyle, aryle, arylalkyle, C0₂ alkyle, COR₂ dans lequel R₂ représente un alkyle, alcynyle, ou aryle, arylalkyle ou hétérocycle.
Z représente :
   - soit hydrogène,
   - soit 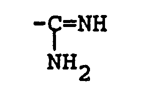
   - soit 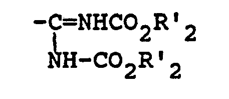 dans lequel R₂ représente un alkyle, arylalkyle,
   - soit CO₂ alkyle,
   - soit CO₂ arylalkyle,
   - soit COR"₂ dans lequel R"₂ peut prendre une des valeurs de R₂,
   - soit -CH₂R₃ dans lequel R₃ représente un aryle, hétéroaryle substitué, hétérocycle, ou peut prendre les valeurs indiquées pour R₁,
   - soit -COCH₂R₃ dans lequel R'₃ peut prendre une des valeurs de R₃.

Les produits de formule (I) présentent des propriétés fongicides.

## Description

La présente invention concerne de nouvelles polyamines acylées, leur procédé de préparation et leur application comme fongicides.

L'invention a pour objet les composés de formule générale (I) : 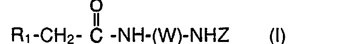 dans laquelle,
R, représente:
   - soit un radical ArO- dans lequel Ar représente un radical aryle renfermant jusqu'à 14 atomes de carbone éventuellement substitué ou un radical hétéroaryle éventuellement substitué, ou un radical hétérocyclique éventuellement substitué,
   - soit un radical aryle, ou polyaryle condensé ou non ou un radical hétérocyclique éventuellement substitué.
W représente :
   - soit un radical (CH₂)ₙ₁ dans lequel ni représente un nombre entier pouvant varier de 2 à 6,
   - soit un radical (CH₂)n₂ NY(CH₂)n₃ dans lequel n₂ et n₃ identiques ou différents représentent un nombre entier pouvant varier de 2 à 6 et Y représente un atome d'hydrogène ou un radical alkyle renfermant jusqu'à 12 atomes de carbone ou un radical aryle renfermant jusqu'à 14 atomes de carbone éventuellement substitué ou un radical arylalkyle renfermant jusqu'à 18 atomes de carbone éventuellement substitué, ou un radical C0₂ alkyle dans lequel le radical alkyle renferme jusqu'à 12 atomes de carbone, ou un radical COR₂ dans lequel le radical R₂ représente un radical alkyle, alcényle ou alcynyle renfermant jusqu'à 12 atomes de carbone ou un radical aryle renfermant jusqu'à 14 atomes de carbone ou un radical arylalkyle renfermant jusqu'à 18 atomes de carbone éventuellement substitué ou un radical hétérocyclique éventuellement substitué et
Z représente :
   soit un atome d'hydrogène,
   - soit un radical 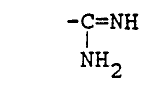
   - soit un radical 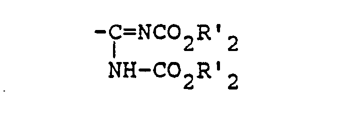 dans lequel R'₂ représente un radical alkyle renfermant jusqu'à 12 atomes de carbone, ou un radical arylalkyle renfermant jusqu'à 18 atomes de carbone,
   - soit un radical CO₂ alkyle dans lequel le radical alkyle renferme jusqu'à 12 atomes de carbone,
   - soit un radical CO₂ arylalkyle dans lequel le radical arylalkyle renferme jusqu'à 18 atomes de carbone,
   - soit un radical COR"₂ dans lequel R"₂ peut prendre l'une des valeurs indiquées ci-dessus pour R₂,
   - soit un radical -CH₂R₃ dans lequel R₃ représente un radical aryle éventuellement substitué, hétéroaryle éventuellement substitué, hétérocyclique éventuellement substitué ou peut prendre les valeurs indiquées ci-dessus pour R,,
   - soit un radical -COCH2R ₃ dans lequel R'₃ peut prendre l'une des valeurs indiquées ci-dessus pour R₃, à la condition que Z ne représente pas un atome d'hydrogène, si W représente un radical (CH₂)₃ et R₁ représente un radical 2,4-dichlorophénoxy ainsi que les sels d'addition avec les acides organiques ou minéraux des composés de formule (I).

L'invention a notamment pour objet les composés de formule (I') : 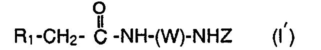 dans laquelle,
Ri représente :
   - soit un radical ArO- dans lequel Ar représente un radical aryle renfermant jusqu'à 14 atomes de carbone éventuellement substitué ou un radical hétéroaryle éventuellement substitué, ou un radical hétérocyclique éventuellement substitué,
   - soit un radical aryle, ou polyaryle condensé ou non ou un radical hétérocyclique éventuellement substitué. W représente :
   - soit un radical (CH₂)nₗ dans lequel n, représente un nombre entier pouvant varier de 3 à 6,
   - soit un radical (CH₂)n₂ NY(CH₂)n₃ dans lequel n₂ et n₃ identiques ou différents représentent un nombre entier pouvant varier de 2 à 6 et Y représente un atome d'hydrogène ou un radical alkyle renfermant jusqu'à 12 atomes de carbone ou un radical aryle renfermant jusqu'à 14 atomes de carbone éventuellement substitué ou un radical arylalkyle renfermant jusqu'à 18 atomes de carbone éventuellement substitué, ou un radical C0₂ alkyle dans lequel le radical alkyle renferme jusqu'à 12 atomes de carbone, ou un radical COR₂ dans lequel le radical R₂ représente un radical alkyle, alcényle ou alcynyle renfermant jusqu'à 12 atomes de carbone ou un radical aryle renfermant jusqu'à 14 atomes de carbone ou un radical arylalkyle renfermant jusqu'à 18 atomes de carbone éventuellement substitué ou un radical hétérocyclique éventuellement substitué et
Z représente :
   - soit un atome d'hydrogène,
   - soit un radical 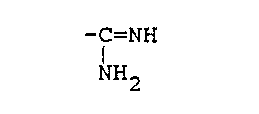
   - soit un radical 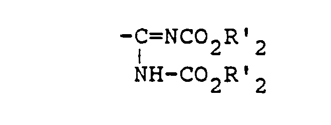 dans lequel R'₂ représente un radical alkyle renfermant jusqu'à 12 atomes de carbone, ou un radical arylalkyle renfermant jusqu'à 18 atomes de carbone,
   - soit un radical C0₂ alkyle dans lequel le radical alkyle renferme jusqu'à 12 atomes de carbone,
   - soit un radical C0₂ arylalkyle dans lequel le radical arylalkyle renferme jusqu'à 18 atomes de carbone,
   - soit un radical -CH₂R₃ dans lequel R₃ représente un radical aryle éventuellement substitué, hétéroaryle éventuellement substitué, hétérocyclique éventuellement substitué ou peut prendre les valeurs indiquées ci-dessus pour Ri,
   - soit un radical -COCH₂R₃ dans lequel R'₃ peut prendre l'une des valeurs indiquées ci-dessus pour R₃, à la condition que Ri ne représente ni un radical 2,5-dihydro xyphényle, ni un radical 2,5-diméthoxyphényle et que Z ne représente pas un atome d'hydrogène, si W représente un radical (CH₂)₃ et Ri représente un radical 2,4-dichlorophénoxy ainsi que les sels d'addition avec les acides organiques ou minéraux des composés de formule (I).

Parmi les sels d'addition avec les acides, on peut citer ceux formés avec les acides minéraux tels que les acides chlorhydrique, bromhydrique, iodhydrique, nitrique ou phosphorique, ou avec les acides organiques comme l'acide formique, acétique, trifluoroacétique, propionique, benzoïque, maléïque, fumarique, succinique, tartrique, citrique, oxalique, glyoxylique, aspartique, alcanesulfoniques tels que les acides méthane ou éthane sulfoniques, ou arylsulfoniques tels que les acides benzène ou paratoluène sulfoniques.

Dans la définition des différents substituants :
- aryle représente de préférence, un radical phényle,
- polyaryle représente de préférence, un radical naphtyle ou un radical biphényle,
- alkyle représente de préférence, un radical méthyle, éthyle, n-propyle, isopropyle, n-butyle, iso-butyle, sec-butyle ou tert-butyle,
- alcényle représente de préférence, un radical vinyle, allyle ou 1,1-diméthylallyle.
- alcynyle représente de préférence un radical éthynyle ou propynyle.
- Les radicaux hétérocycliques concernés sont de préférence les radicaux pyridyle, furanyle, thiophényle, indolyle, coumarinyle, oxazolyle, thiazolyle ou un radical 7-(3,7-dihydro 1,3-diméthyl 2,6-dioxo) 1 H-purinyle.
- Les radicaux arylalkyles des radicaux W et Z, lorsqu'ils sont substitués le sont de préférence par des radicaux hydroxyle, des atomes d'halogène comme par exemple des atomes de chlore ou de brome, des radicaux CF₃, des radicaux alkyloxy renfermant jusqu'à 4 atomes de carbone par exemple le radical méthoxy.

L'invention a plus particulièrement pour objet les composés de formule (I) dans lesquels les radicaux aryle, hétéroaryle, arylalkyle et hétérocyclique sont éventuellement substitués par un ou plusieurs substituants choisis dans le groupe constitué par les radicaux hydroxyle, les atomes d'halogène, les radicaux alkyle, alcényle et alcynyle renfermant jusqu'à 12 atomes de carbone, les radicaux aryle renfermant jusqu'à 14 atomes de carbone, les radicaux CF₃, les radicaux OR"3 dans lequel R"₃ représente un radical alkyle renfermant jusqu'à 12 atomes de carbone ou un radical arylalkyle renfermant jusqu'à 18 atomes de carbone, les radicaux coalkyle, Si(alkyle)₃, S0₂alkyle dans lesquels le radical alkyle renferme jusqu'à 12 atomes de carbone, et les radicaux S0₂aryle dans lesquels le radical aryle renferme jusqu'à 14 atomes de carbone, les différents radicaux pouvant former entre eux avec les atomes auxquels ils sont liés des cycles 0-CH₂-0, ainsi que leurs sels d'addition avec les acides organiques ou minéraux.

Parmi les composés préférés de l'invention, on peut citer les composés dans lesquels W représente un radical -(CH₂)₃-(CH₂)₄- ou (CH₂)₅- ou un radical (CH₂)n₄NH(CH₂)ns dans lequel n4 et ns identiques ou différents représentent les nombres 3, 4 ou 5 ainsi que leurs sels d'addition avec les acides organiques ou minéraux.

L'invention a notamment pour objet les composés de formule (I) dans lesquels Z représente un atome d'hydrogène ou un radical C0₂alkyle, dans lequel le radical alkyle renferme jusqu'à 12 atomes de carbone ou un radical 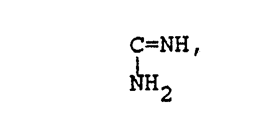 ou un radical 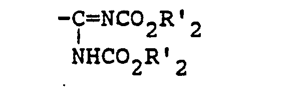 R'₂ gardant la même signification que précédemment ainsi que leurs sels d'addition avec les acides organiques ou minéraux.

L'invention a particulièrement pour objet les composés de formule (I) dans lesquels Ri représente un radical ArO- dans lequel Ar représente un radical aryle éventuellement substitué comme par exemple le radical : 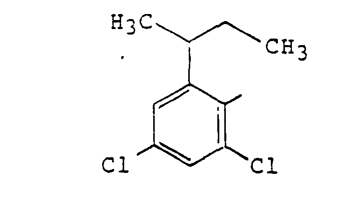 ainsi que leurs sels d'addition avec les acides organiques ou minéraux.

Parmi les composés préférés de l'invention, on peut également citer les composés dans lesquels R, représente un radical : 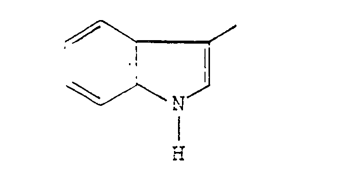 un radical : 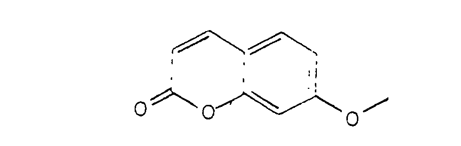 ou un radical : 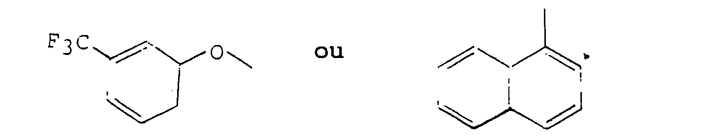 ainsi que leurs sels d'addition avec les acides organiques ou minéraux.

Parmi les composés préférés de l'invention, on peut citer tout particulièrement les produits dont la préparation est donnée ci-après dans la partie expérimentale et tout particulièrement les produits dont les noms suivent :
- N-[(6-sec-butyl 2,4-dichloro phénoxy) acétyl] 1,4-diamino butane,
- 3,3'-[N-(6-sec-butyl 2,4-dichlorophénoxy) acétyl] 3,3'-diaminodipropylamine,
- N-(7-acétoxy coumarinyl) 1,4-diamino butane,
- N-[(6-sec-butyl 2,4-dichlorophénoxy) acétyl] NN'-spermidine,
- N-[(6-sec-butyl 2,4-dichlorophénoxy) acétyl] 4-guanidine amino butane,
- N-[(6-sec-butyl 2,4-dichlorophénoxy) acétyl] diamino 1,3-propane,
- N-[(6-sec-butyl 2,4-dichlorophénoxy) acétyl] diamino 1,5-pentane, ainsi que leurs sels d'addition avec les acides organiques ou minéraux.

L'invention a également pour objet un procédé de préparation des composés de formule (I) caractérisé en ce que l'on soumet un acide de formule (II) :
R₁CH₂CO₂H (II)
   dans laquelle R, conserve sa signification précédente ou un dérivé fonctionnel de cet acide, à l'action d'une polyamine de formule (III) :
NH₂-W-NH-Z (III)

dans laquelle W et Z conservent la même signification que précédemment, pour obtenir le composé de formule (I) correspondant, que l'on soumet si désiré dans le cas où Z est un atome d'hydrogène à l'action d'un agent de fonctionnalisation de la fonction amine, puis soumet si désiré le composé de formule (I) obtenu à l'action d'un acide pour en former le sel.

Cette acylation directe de l'amine NH₂(W)NHZ par l'acide R₁CH₂CO₂H peut se faire directement par chauffage ou par activation de la fonction acide à l'aide par exemple de dicyclohexylcarbodiimide, de N,N'- carbonyldiimidazole ou de diéthylcyanophosphonate ou encore par l'intermédiaire des dérivés de l'acide selon la réaction :
R₁CH₂COL+NH₂-(W)-NH-Z→ 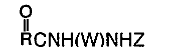 L pouvant prendre l'une des valeurs suivantes : L = CI ou OCOR' L = 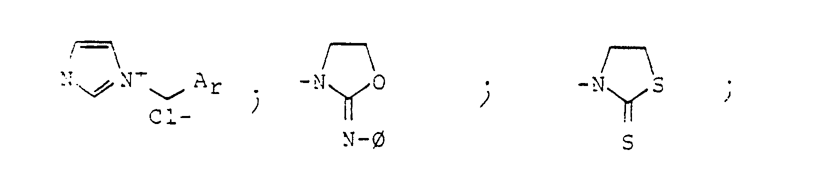 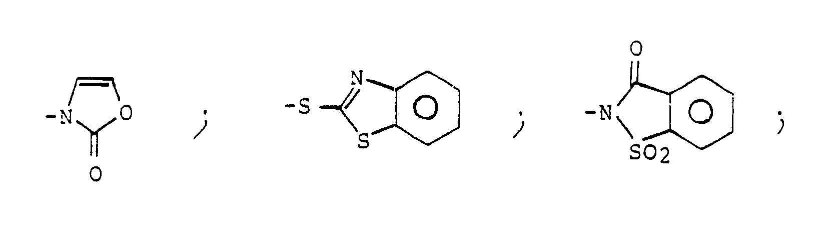 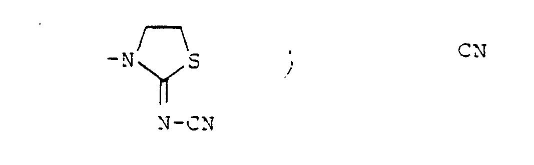 Voir à ce sujet les travaux de :
J. March "Advanced. Organic Chemistery" Sec. Ed p 384
Albertson Org. React. 12, 205-218 (1962)
Klausner, Bodansky, Synthesis 453 (1972)
Paul, Anderson, J. Am. Chem. Soc 82, 4596, (1960)
Paul, Anderson, Tetrahedron Lett. 1595 (1973)
Paul, Anderson, Tetrahedron 32, 2211 (1976)
Beckwit "Chemistery of Amides" Ed. Zabicky 73-185 (1970)
Sonntag, Chem. Rev 52, 237-416 (1953)
Sonntag, Chem. Pharm. Bull (1982) 30, 4242
Sonntag, Bull. Chim. Soc. Fr (1982) II, 167
Sonntag, Tetrahedron Lett. (1980) 21, 841
Sonntag, Tetrahedron Lett. (1985) 26, 1977
Sonntag, Synthesis (1982) 933
Sonntag, Chem. Lett. (1985) 123
Sonntag, Heterocycles (1988) 27, 323
Sonntag, Chem. Lett. (1987) 879-882..

Les composés de formule (III) sont connus de façon générale et peuvent être préparés de façon classique selon les procédés décrits par Green Protective Groups in Organic Synthesis Wiley Ed, Hoppe Seyler's Physiol. Chem. 357, 1651 (1976), Bergeron et All Synthesis 1982, 689, Bergeron et All J. Org. Chem. (49) 2997, Atwell et Coll Synthesis 1984 (1033). Certains produits de formule (III) dans lesquels W représente le radical -(CH₂)n₄NH(CH₂)n_{S}- dans lesquels n₄ et ns sont définis comme précédemment ainsi que ceux pour lesquels 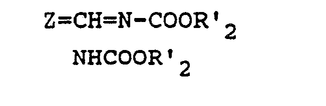 et W=(CH₂)ₙ avec n variant de 2 à 6, sont des produits nouveaux et sont un des objets de la présente invention, leur préparation est donnée ci-après dans la partie expérimentale.

L'invention a également pour objet un procédé dans lequel R₁ est un radical ArO- caractérisé en ce que l'on soumet un composé de formule (VI) :
ArOH (VI)
à l'action d'un composé de formule : 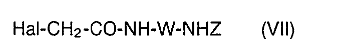 dans laquelle W et Z conservent leur signification précédente et Hal représente un atome d'halogène pour obtenir le composé de formule (lₛ) correspondant : 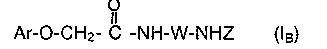 que l'on soumet si désiré à l'action d'un acide pour en former le sel.

Les composés de formule (VII) utilisés comme produits de depart sont des produits nouveaux et sont en eux-mêmes un objet de la présente invention, leur préparation est donnée ci-après dans la partie expérimentale. Elle peut être résumée par les schémas suivants :

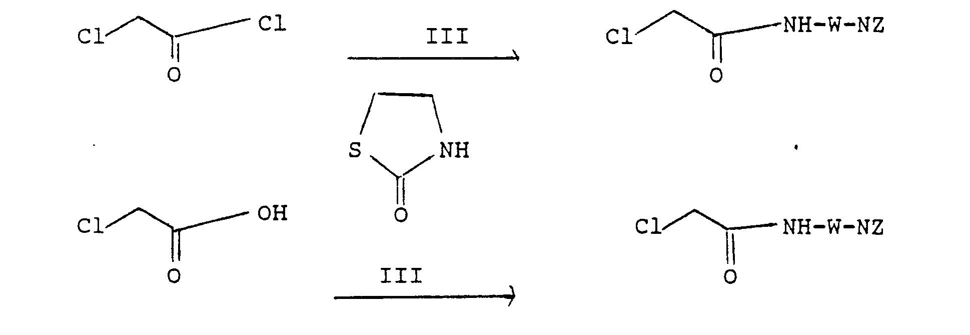

Les composés de formule (I) présentent d'intéressantes propriétés fongicides susceptibles d'être utilisées pour la protection vis-à-vis des champignons pathogènes. Il peut s'agir de la protection des plantes, de la protection des locaux ou des animaux.

Ces propriétés peuvent également être utilisées en hygiène et médecine humaine et animale.

Les composés de l'invention permettent de lutter contre de très nombreux champignons phytopathogè- nes, notamment contre :
Erysiphe graminis, Sphaerotheca macularis, Sphaerotheca fuliginea, Podosphaera leucotricha, Uncinula necator, Helminthosporium spp., Rhynchosporium spp., Septoria spp., Pseudocercosporella herpotrichoides and Gaeumannomyces graminis, Ustilago spp., Cercospora arachidicola and Cercosporidium personatum, Cercospora species, Botrytis cinerea, Alternaria spp., Venturia inaequalis, Plasmopara viticola, Bremia lactucae, Peronospora spp., Pseudoperonospora humuli, Pseudoperonospora cubensis, Phytophthora spp. infestans, Phytophthora spp., Thanatephorus cucumeris, Rhizoctonia spp. ou encore des champignons ou levures intéressant la santé humaine comme Candida albicans ou Trychophyton spp.

L'invention a donc pour objet les compositions fongicides renfermant comme principe actif au moins un composé de formule (I) tel que défini précédemment. L'invention a plus particulièrement pour objet les composés dont la préparation chimique est donnée aux exemples 1, 3, 6, 7, 8, 20, 21 et 22.

Les compositions selon l'invention sont préparées selon les procédés usuels de l'industrie agrochimique par exemple.

Ces compositions peuvent se présenter sous forme de poudres, granulés, suspensions, émulsions, solutions, ou autres préparations employées classiquement pour l'utilisation de ce genre de composés.

Outre le principe actif, ces compositions contiennent, en général, un véhicule et/ou un agent tensioactif, non ionique, assurant, en outre, une dispersion uniforme des substances constitutives du mélange. Le véhicule utilisé peut être un liquide, tel que l'eau, l'alcool, les hydrocarbures ou autres solvants organiques, une huile minérale, animale ou végétale, une poudre telle que le talc, les argiles, les silicates, le kieselguhr ou un solide combustible.

Les compositions peuvent naturellement renfermer un ou plusieurs autres agents pesticides, par exemple un ou plusieurs agents insecticides ou acaricides.

Les exemples suivants illustrent l'invention sans toutefois la limiter.

### EXEMPLE 1 : N-[(6-sec-butyl 2,4-dichloro phénoxy) acétyl] 1,4-diamino butane

### Stade A : N-(tert-butoxycarbonyl) N -[(6-sec-butyt 2,4-dichloro phénoxy) acétyl] 1,4-diamino butane.

A un mélange de 3,95 g de N-(tert-butoxycarbonyl) 1,4-diamino butane (préparation 1), 40 cm³ de chloroforme et 3 cm³ de triéthylamine, on ajoute à + 10. C cm³ suspension de 6,2 g de chlorure d'acide (6- sec-butyl 2,4-dichloro phénoxy) acétique obtenue au stade D de la préparation ci-après et 50 cm³ de chloroforme. On agite pendant 1 heure à température ambiante puis évapore à sec sous pression réduite.

On reprend le résidu avec du tétrahydrofuranne, filtre l'insoluble, amène le filtrat à sec sous pression réduite. On chromatographie le résidu (10,15 g) sur silice (éluant : hexane-acétate d'éthyle (7-3)), on obtient 8,42 g de produit attendu. F - 50 ° C.

### Stade B : N-[(6-sec-butyl 2,4-dichloro phénoxy) acétyl] 1,4-diamino butane.

### a) Obtention du chlorhydrate :

On agite au reflux pendant 1 heure trente 8,12 g du produit obtenu au stade A, 80 cm³ d'éthanol et 20,1 cm³ d'éthanol chlorhydrique -- 2N. On concentre sous pression réduite, on lave le résidu au chlorure de méthylène. On obtient 6,7 g de chlorhydrate attendu.

### b) Obtention de la base :

On agite 6,7 g de chlorhydrate avec 100 cm³ d'éthanol et 3,2 g de bicarbonate de sodium pendant 4 heures au reflux. On filtre à chaud et concentre le filtrat sous pression réduite. On chromatographie le résidu (6,05 g) sur silice (éluant : méthanol-ammoniaque (98-2)), on obtient 4,74 g du produit recherché.

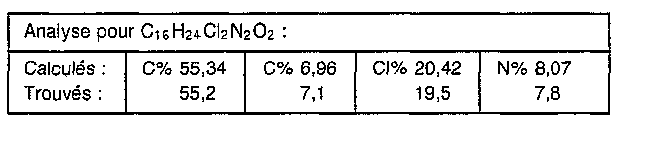

Préparation de chlorure de l'acide (6-sec-butyl 2,4-dichloro phénoxy) acétique utilisé au départ de l'exemple 1.

### Etape A : 6-sec-butyl 2,4-dichloro phénol

On agite pendant 1 heure 30 au reflux, 62,76 g de 6-sec-butyl phénol et 75 cm³ de chlorure de sulfuryle. On ramène à température ambiante et verse sur 400 cm³ d'eau, extrait avec du chlorure de méthylène et évapore à sec sous pression réduite. L'huile obtenue (89,8 g) est distillée à deux reprises Eb 82 C sous 0,01 mm de mercure, on obtient 27,8 g de produit attendu.

### Etape B : 6-sec-butyl 2,4-dichloro phénoxy acétate d'éthyle.

A un mélange de 5 g du produit obtenu à l'étape A et 20 cm³ de diméthyl sulfoxyde, on ajoute à température ambiante 2,8 cm³ de bromoacétate d'éthyle. A la solution obtenue, on ajoute par portions, 4,45 g de carbonate de potassium puis chauffe ensuite pendant 3 heures à 70/80 C. On verse sur un mélange eau et glace, extrait avec du chlorure de méthylène et évapore à sec sous vide. On chromatographie le résidu (7,13 g) sur silice (éluant : hexane-acétate d'éthyle (97-3)).
On obtient 6,58 g de produit attendu.

### Etape C : Acide 6-sec-butyl 2,4-dichloro phénoxy acétique.

On agite pendant 1 heure à 20°C, 6,5 g du produit obtenu à l'étape B, 15 cm³ d'éthanol et 10,65 cm³ de soude 2N. On ajoute ensuite 10,65 cm³ d'acide chlorhydrique 2N et évapore à sec sous pression réduite, le résidu (10 g) est repris avec 200 cm³ d'eau. On essore, sèche et obtient 5,83 g de produit attendu.

### Etape D : Chlorure de l'acide 6-sec-butyl 2,4-dichloro phénoxy acétique.

On agite au reflux, pendant deux heures, 5,8 g du produit obtenu à l'étape C avec 40 cm³ de chlorure de thionyle. On évapore à sec sous pression réduite, lave l'huile obtenue avec du benzène et évapore à sec sous pression réduite.

On obtient 6,2 g de produit attendu utilisé tel quel pour l'obtention du produit de l'exemple 1.

### EXEMPLE 2 : N-[1-(3-indolyl) acétyl] 1,4-diamino butane

### Stade A : N-(tert-butoxycarbonyl) N'-[1-(3-indolyl) acétyl] 1,4-diamino butane.

On opère comme au stade A de l'exemple I à partir de 8,10 g de N-(tert-butoxycarbonyl) 1,4-diamino butane (préparation 1) et 8,39 g de chlorure de 1,3-indol acétyle (obtenu suivant préparation décrite ci-après), dans le chlorure de méthylène. L'extrait sec est repris par du tétrahydrofuranne, on filtre l'insoluble, évapore à sec et obtient 16,53 g de produit que l'on chromatographie sur silice (éluant : chloroforme méthanol (98-2) puis à nouveau en éluant acétate d'éthyle-hexane (7-3) et enfin à l'acétate d'éthyle).
On obtient 11,47 g de produit attendu.

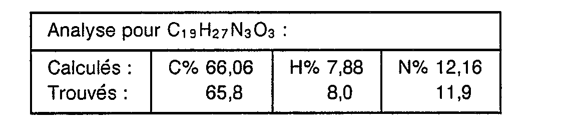

### Stade B : N-[1-(3-indolyl) acétyl] 1,4-diamino butane.

On opère comme au stade B de l'exemple 1 en utilisant 9,35 g du produit obtenu au stade A et 35 cm³ d'éthanol chlorhydrique 1,6 N. On obtient après une double chromatographie 2,28 g de produit attendu.

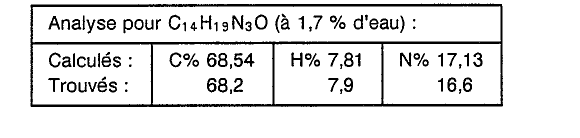

### Préparation de chlorure de 1,3-indolacétyle, utilisé au départ de l'exemple 2.

A une suspension agitée à 0 C de 27 g de pentachlorure de phosphore et 500 cm³ d'éther éthylique, on ajoute lentement 20 g d'acide indole acétique, on agite encore 15 minutes à 0°C. On concentre ensuite sous pression réduite jusqu'à un volume total de 200 cm³ puis ajoute 2 litres d'hexane, agite 2 heures, essore et sèche sous pression réduite.
On obtient 8,38 g de produit attendu. F = 66 C.

### EXEMPLE 3 : Chlorhydrate de N-(7-acétoxy coumarinyl) 1,4-diamino butane

### Stade A : N-(tert-butoxycarbonyl)N - (7-acétoxy coumarinyl) 1,4-diamino butane.

A une solution de 10 g de 7-hydroxy coumarine dans 100 cm3 de diméthyl formamide, on ajoute à température ambiante 2,96 g d'hydrure de sodium à 50 % dans l'huile. On agite pendant 45 minutes à température ambiante et on ajoute 17,98 g de chloracétyl N-(tert-butoxycarbonyl) 1,4-diamino butane (préparation ci-après) en solution dans 60 cm³ de diméthyl formamide, on agite 5 heures à 90°C puis 60 heures à température ambiante. On évapore sous pression réduite. On reprend le résidu (37,6 g) dans 180 cm³ d'eau, essore, empâte éther isopropylique. On obtient 23,25 g de produit recherché. F = 120°C.

### Stade B : Chlorhydrate de N-(7-acétoxy coumarinyl) 1,4-diamino butane.

On agite 1 heure au reflux 19,85 g de produit obtenu au stade A et 38,1 cm³ d'une solution d'éthanol chlorhydrique 2N dans 200 cm³ d'éthanol. On évapore à sec, on obtient 18,67 g de produit brut. F = 90 C.

On recristallise dans 20 ml de mélange acétonitrile-eau (95-5) au reflux, essore, empâte éther isopropylique. On obtient 8,44 g de produit attendu. F = 160 C.

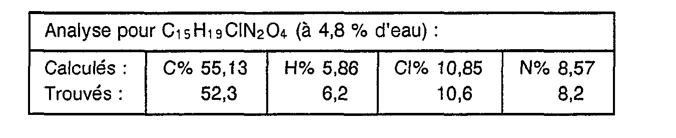

### Préparation de chloracétyl N-(tert-butoxycarbonyl) 1,4-diamino butane utilisé pour l'exemple 3.

A un mélange agité à 0°C de 9,4 g de N-(tert-butoxycarbonyl) 1,4-diamino butane (préparation 1) et 7,3 cm³ de triéthylamine dans 100 cm³ de tétrahydrofuranne, on ajoute à 0°C une solution de 5,65 g de chlorure d'acide chloracétique dans 50 cm³ de tétrahydrofuranne. On agite 2 heures à température ambiante. On évapore à sec et reprend le résidu avec 100 cm³ de chlorure de méthylène et 50 cm³ d'eau, on décante et concentre la fraction organique à sec, sous pression réduite, on obtient 12 g de produit attendu. F = 98-100 °C.

### EXEMPLE 4 : Chlorhydrate de N-(3-trifluorométhyl phénoxy acétyl) 1,4-diamino butane

### Stade A : N-(tert-butoxycarbonyl) N'-(3-trifluorométhyl phénoxy acétyl) 1,4-diamino butane.

A une solution, refroidie à +10°C, de 10,2 g de N-(tert-butoxycarbonyl) 1,4-diamino butane (préparation 1) dans 150 cm³ de chloroforme, on ajoute 6,44 g de chlorure d'acide trifluorométhyl phénoxy acétique (préparation ci-après) en solution dans 60 cm³ de chloroforme. On agite deux heures à température ambiante. On concentre sous pression réduite, reprend à l'eau, essore et on obtient: 9,85 g de produit recherché. F = 86 C.

### Stade B : Chlorhydrate de N-(3-trifluorométhyl phénoxy acétyl) 1,4-diamino butane.

On agite pendant 1 heure au reflux, 9,8 g de N-(tert-butoxycarbonyl) N'-(3-trifluorométhyl phénoxy acétyl) 1,4-diamino butane obtenu au stade A, 90 cm³ d'éthanol et 28 cm³ d'éthanol chlorhydrique 2N. On concentre à sec sous pression réduite, on obtient 11,9 g de produit que l'on cristallise dans l'acétate d'éthyle, on recueille 7,55 g de produit attendu. F = 90°C.

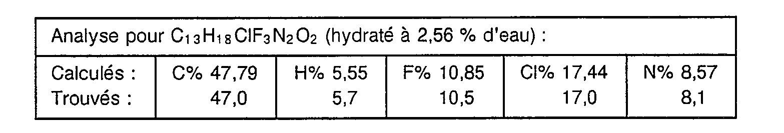

### Préparation du chlorure de l'acide 3-trifluorométhyl phénoxy acétique, utilisé au départ de l'exemple 4.

### Etape A : 3-trifluorométhyl phénoxy acétate d'éthyle.

A un mélange de 15 cm³ de 3-trifluorométacresol et 60 cm³ de diméthylsulfoxyde, on ajoute 15 cm³ de bromoacétate d'éthyle puis par portions 24 g de carbonate de potassium. On chauffe cinq heures à 70-80 C, puis on coule sur 400 cm³ d'un mélange d'eau et glace. On extrait avec du chlorure de méthylène, on évapore à sec sous pression réduite, reprend le résidu avec de la soude diluée, extrait avec du chlorure de méthylène et concentre à sec sous pression réduite, on obtient 28,61 g de produit attendu utilisé tel quel pour l'étape suivante.

### Etape B : Acide 3-trifluorométhyl phénoxy acétique.

On agite pendant 1 heure une solution de 23,3 g de produit obtenu à l'étape A dans 50 cm³ d'éthanol et 47 cm³ de soude 2N. On ajoute ensuite 47 cm³ d'acide chlorhydrique 2N. On concentre à sec sous pression réduite, reprend le résidu à l'eau et essore. On obtient 19,73 g de produit attendu. F = 96^{.} C.

### Etape C : Chlorure de l'acide 3-trifluorométhyl phénoxy acetique.

On agite 1 heure au reflux, une solution de 6 g de l'acide 3-trifluorométhyl phénoxy acétique (obtenu à l'étape B) dans 36 cm³ de chlorure de thionyle, on évapore à sec sous pression réduite, on obtient 6,44 g de produit recherché, utilisé tel quel au départ de l'exemple 4.

### EXEMPLE 5 : N-(3-trifluorométhyl phénoxy acétyl) N'-[1-(3-indol acétyl)] 1,4-diamino butane

A 6,44 g d'iodure de 2-chloro N-méthyl pyridinium et 100 cm³ de tétrahydrofuranne, on ajoute 3,72 g d'acide 3-indol acétique puis lentement une solution de 6 g de 3-trifluorométhyl phénoxy acétyl 1,4-diamino butane (obtenu au stade B de l'exemple 4) dans 150 cm³ de tétrahydrofuranne, puis ajoute 7 cm³ de triéthylamine et on agite pendant 6 heures au reflux et 16 heures à température ambiante. On évapore à sec sous pression réduite et chromatographie le résidu (16,7 g) sur silice (éluant : chlorure de méthylène- tétrahydrofuranne (1-1)), on obtient 6,19 g de produit attendu.

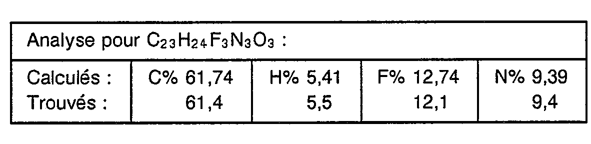

### EXEMPLE 6 : Dichlorhydrate de 3,3'-[N-(6-sec-butyl 2,4-dichloro phénoxy) acétyl] 3,3'-diaminodipropylamine

### Stade A : N-[(6-sec-butyl 2,4-dichloro phénoxy) acétyl] thiazolidine-2-thione.

A une solution de 9,78 g d'acide (6-sec-butyle 2,4-dichloro phénoxy) acétique (préparé comme à l'étape C de la préparation utilisée à l'exemple 1), 4,63 g de 2-mercaptothiazoline dans 80 cm³ de chlorure de méthylène, on ajoute à -5/0°C, une solution de 8,09 g de dicyclohexylcarbodiimide, 340 mg de diméthylamino pyridine dans 60 Cm₃ de chlorure de méthylène. On agite 10 minutes à 0°C et quatre heures à température ambiante. On filtre l'insoluble, amène à sec et on chromatographie le résidu sur silice (éluant : hexane-acétate d'éthyle (7-3)), on obtient 10,3 g de produit attendu.
F = 96°C.

### Stade B : 3,3'-[N-[(6-sec-butyl 2,4-dichloro phénoxy) acétyl] N"-(tert-butoxycarbonyl)] 3,3'-diaminodipropylamine.

A une solution de 1,59 g de produit obtenu selon la préparation ci-après, dans 15 cm³ de chlorure de méthylène, on ajoute à 0°C une solution de 2,6 g de produit obtenu au stade A dans 25 cm³ de chlorure de méthylène. On agite 45 minutes à température ambiante, amène à sec et chromatographie sur silice (éluant : méthanol), on obtient 3,19 g de produit attendu.

### Stade C : Dichlorhydrate de 3,3'-[N-(6-sec-butyl 2,4-dichloro phénoxy) acétyl] 3,3'-diaminodipropylamine.

A une solution de 3,19 g de produit obtenu au stade B dans 30 cm³ d'éthanol, on ajoute 6,4 cm³ d'une solution 2,25 N d'éthanol chlorhydrique, on chauffe 1 heure au reflux, amène à sec sous pression réduite et empâte le résidu dans l'éther isopropylique. On obtient 2,58 g de produit attendu. F = 196°C.

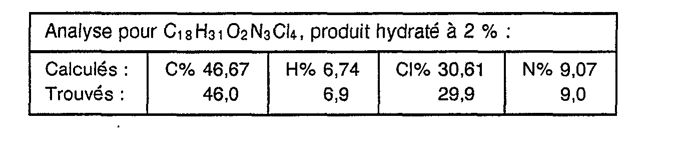

### Préparation de N-(tert-butoxycarbonyl) (3-amino propyl) 1,3-propanediamine, utilisé au stade B de l'exemple 6.

A une solution de 39,3 g de 3,3'-diaminodipropylamine dans 300 cm³ de chlorure de méthylène, on ajoute à 5⁰ C : une solution de 33 g de dicarbonate de tert-butyle dans 200 cm³ de chlorure de méthylène. On agite 16 heures à 20 C, on ajoute de l'eau puis décante, évapore à sec et chromatographie sur silice (éluant : méthanol-ammoniaque (98-2)), on obtient 15,5 g de produit attendu, utilisé tel quel pour la préparation du produit de l'exemple 6.

### EXEMPLE 7 : Trifluoroacétate de N-[(6-seo-butyl 2,4-dichloro-phénoxy) acétyl] 4-guanidine aminobutane

### Stade A : N-[(6-sec-butyl 2,4-dichlorophénoxy) acétyl] 4-(di-tert-butoxycarbonyl guanidine) aminobutane.

A une solution de 4,89 g de N-(di-tert-butoxycarbonyl guanidine) aminobutane (préparée comme ci-dessous) dans 50 cm³ de chlorure de méthylène, on ajoute à 0 C, 3,43 g de produit obtenu au stade A de l'exemple 6, en solution dans 40 cm³ de chlorure de méthylène, on agite pendant 1 heure à température ambiante puis amène à sec et on chromatographie le résidu sur silice (éluant : hexane-acétate d'éthyle (7-3) à 1 % de triéthylamine). On obtient 3,63 g de produit attendu.

### Stade B : Trifluoroacétate de N-[(6-sec-butyl 2,4-dichloro phénoxy) acétyl] 4-guanidine aminobutane.

A une solution de 1,82 g de produit obtenu au stade A dans le chlorure de méthylène, on ajoute à température ambiante, 2,4 cm³ d'acide trifluoro acétique. On agite pendant 48 heures et amène à sec sous pression réduite, on obtient 1,56 g de produit attendu.

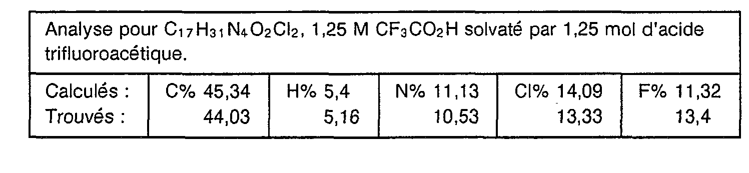

### Préparation de N,-(N'₁,N'₂-bis-tert-butoxycarbonyl formamidino) 1,4-diamino butane, utilisé au départ de l'exemple 7.

### Etape A : N-benzyloxy carbonyle 1,4-diamino butane.

A un mélange de 23 g de 1,4-diamino butane, 50 cm³ d'eau, on ajoute, sans dépasser 30°C, une solution de 46 g d'acide méthane sulfonique dans 50 cm³ d'eau. Le milieu réactionnel est dilué avec 140 cm³ d'éthanol. On ajoute alternativement à pH 3,5-5,0 une solution de 39 g de chloroformiate de benzyle dans 50 cm³ de 1,2-diméthoxy éthane et 100 cm³ d'une solution aqueuse à 50 % d'acétate de potassium. Les additions se font alternativement de manière à maintenir la valeur du pH entre 3,5 et 5,0. On agite pendant encore 1 heure 30 à 20-25 °C et évapore sous pression réduite. On verse le résidu sur 500 cm³ d'eau, filtre l'insoluble, on lave le filtrat avec 3 fois 150 cm³ de benzène puis on ajoute 100 cm³ de soude à 30 % saturée avec du chlorure de sodium. On extrait avec du benzène, évapore à sec sous pression réduite, on obtient 19, 10 g de produit attendu.

### Etape B : N,N'-bis-(tert-butoxycarbonyl) méthyl thioisourée.

On agite pendant 48 heures à 20°C. 58,1 g de tert-butyle dicarbonate, 25,3 g de sulfate de méthyl thioisourée, 50 g de bicarbonate de sodium, 500 cm³ d'eau et 500 cm³ de chlorure de méthylène. On décante, extrait avec du chlorure de méthylène évapore à sec. On obtient 27 g de produit attendu. F = 118°C.

### Stade C : Ni -(N'₁,N'₂-bis-tert-butoxycarbonyl formamidino) N₂-carbobenzyloxy 1,4-diamino butane.

On agite pendant 2 heures au reflux un mélange de 13,4 g de produit obtenu à l'étape A, 14,5 g de N,N'-bis-(tert-butoxycarbonyl) méthyl thioisourée obtenue à l'étape B et 100 cm³ de tétrahydrofuranne. On concentre sous pression réduite. On reprend le résidu avec 200 cm³ de chloroforme, lave avec 30 cm³ d'une solution saturée de bicarbonate de sodium dans l'eau et concentre à sec sous pression réduite. On chromatographie sur silice (éluant : acétate d'éthyle). On obtient 12,7 g de produit attendu.

### Etape D : Ni -(N'₁,N'₂-bis-tert-butoxycarbonyl formamidino) 1,4-diamino butane.

A une solution de 12,7 g de produit obtenu au stade C dans 100 cm³ d'éthanol, on ajoute 1 g de charbon actif à 10 % de palladium et agite sous pression d'hydrogène jusqu'à fin d'absorption. On filtre le catalyseur et amène à sec le filtrat sous pression réduite.
On obtient 6,6 g de produit attendu.

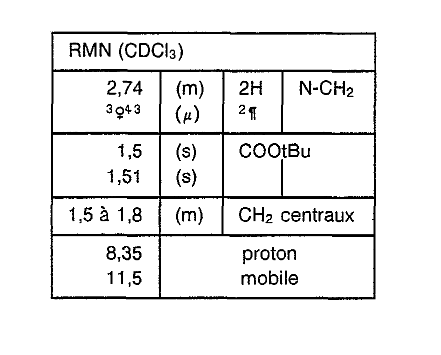

### EXEMPLE 8 : Dichlorhydrate de N-[(6-sec-butyl 2,4-dichloro phénoxy) acétyl] NN'-spermidine

### Stade A : N-[(6-sec-butyl 2,4-dichloro phénoxy) acétyl] NN'-(tert-butoxycarbonyl) spermidine.

On opère comme au stade B de l'exemple 6 en utilisant 1,62 g de NN'-tert-butoxycarbonyl spermidine (préparation ci-après) et 2,5 g de N-[(6-sec-butyl 2,4-dichloro phénoxy) acétyl] thiazolidine-2-thione, préparé au stade A de l'exemple 6. On obtient 2,64 g de produit attendu.

### Stade B : Dichlorhydrate N-[(6-sec-butyl 2,4-dichloro phénoxy) acétyl] NN'-spermidine.

On opère comme au stade C de l'exemple 6. On obtient 2,23 g de produit attendu.

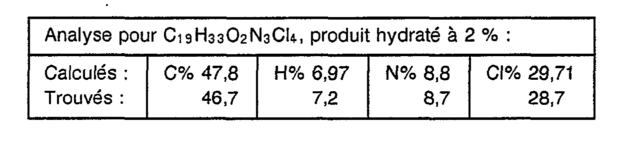

### Préparation de N-(tert-butoxycarbonyl) spermidine, utilisée au stade A de l'exemple 8.

### Etape A : N-phtaloyl aminobutyraldéhyde diéthylacétal.

A une solution de 30,1 g de N-carbéthoxy phtalimide dans 150 cm³ de tétrahydrofuranne, on ajoute à 20 C, une solution de 24,8 g de 4-aminobutyraldéhyde diéthylacétal dans 250 cm³ de tétrahydrofuranne. On agite pendant 3 heures au reflux. On évapore le solvant et chromatographie le résidu sur silice (éluant) : hexane-acétate d'éthyle (65-35)), on obtient 34,9 g de produit recherché.

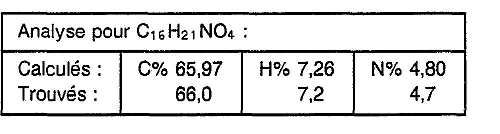

### Etape B : 4-phtalimido butanal.

On agite pendant 3 heures 33,9 g de produit obtenu à l'étape A dans 350 cm³ d'acide chlorhydrique 2N, on dilue avec de l'eau et extrait avec du chlorure de méthylène. On évapore à sec et on obtient 24 g de produit attendu. F = 70° C.

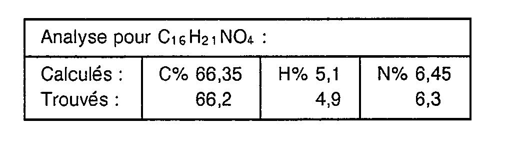

### Etape C : N'-(phtaloyl) N"-(tert-butoxycarbonyl) spermidine.

A une solution de 8,68 g de produit obtenu à l'étape B, 7 g de N-(tert-butoxycarbonyl) 1,3-diamino propane (préparation 2) dans 120 cm³ d'éthanol, on ajoute 2,4 g de charbon palladié et agite sous atmosphère d'hydrogène jusqu'à fin d'absorption. On filtre le catalyseur, évapore le filtrat à sec et chromatographie sur silice (éluant : méthanol à 1 % d'ammoniaque). On obtient 8,5 g de produit attendu.

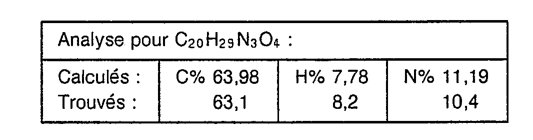

### Etape D : N-(tert-butoxycarbonyl) spermidine.

A une solution de 8,5 g de produit obtenu à l'étape C dans 250 cm³ d'éthanol, on ajoute 4,5 cm³ d'hydrate d'hydrazine à 80 %. On chauffe 1 heure au reflux, filtre l'insoluble et évapore à sec sous pression réduite. On reprend le résidu avec du chlorure de méthylène, lave à l'eau et évapore à sec sous vide. On obtient 4,13 g de produit attendu.

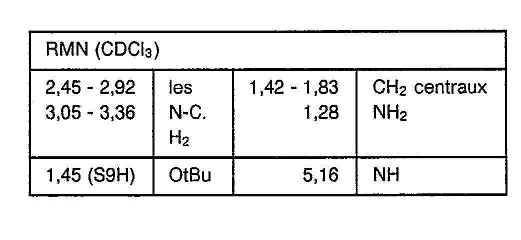

### EXEMPLE 9 : Chlorhydrate de N-(2,4-dichloro phénoxy acétyl) 1,4-diamino butane

### Stade A : N-(tert-butoxycarbonyl) N'-(2,4-dichloro phénoxy acétyl) 1,4-diamino butane.

A un mélange de 8,6 g de N-(tert-butoxycarbonyl) 1,4-diamino butane (préparation 1), 120 cm³ de chlorure de méthylène et 7 cm³ de triéthylamine, on ajoute, sans dépasser 25 C, 11 g de chlorure d'acide 2,4-dichloro phénoxy acétique en solution dans 40 cm³ de chlorure de méthylène. On agite pendant 30 minutes puis concentre à sec sous pression réduite. On reprend le résidu avec 200 cm³ de tétrahydrofuranne, filtre l'insoluble et amène à sec le filtrat sous pression réduite. On recueille 18,53 g de produit brut (F = 107° C) que l'on recristallise dans 740 cm³ d'éther isopropylique. On obtient 17,43 g de produit attendu (F = 110°C). 5,39 g sont chromatographiés sur silice (éluant : hexane-acétate d'éthyle (6-4)), on recueille 4,48 g de produit recherché. F = 114°C.

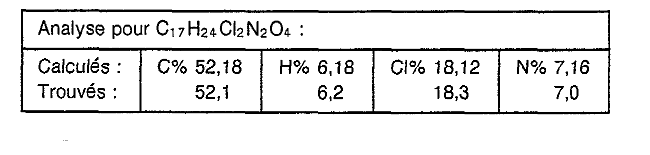

### Stade B : Chlorhydrate de N-(2,4-dichloro phénoxy acétyl) 1,4-diamino butane.

On agite au reflux pendant 2 heures, 10,15 g de produit obtenu comme au stade A, 120 cm³ d'éthanol et 35 cm³ d'une solution 1,5 N d'éthanol chlorhydrique. On concentre sous pression réduite, on recristallise le produit brut obtenu, 12,56 g (F = 90 C), dans 15 volumes d'un mélange éthanol-acétate d'éthyle (1-1), on obtient 5,88 g de produit attendu. F = 140 °C

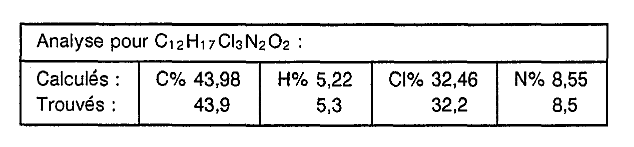

### Préparation de chlorure de 2,4-dichlorophénoxyacétyle utilisé au stade A de l'exemple 9.

A une solution de 130 g d'acide 2,4-dichlorophénoxy acétique dans 150 cm³ de benzène, on ajoute, en 30 minutes, 46 cm³ de chlorure de thionyle, on chauffe ensuite au reflux pendant 1 heure puis élimine le solvant et l'excès de chlorure de thionyle sous pression réduite et distille sous 0,1 mm de mercure. On obtient 91,4 g de produit recherché.
Eb = 120° sous 0,1 mm de mercure.

### EXEMPLE 10 : Dichlorhydrate de 1-(2,4-dichloro phénoxy acétyl) triaza 1,6,10-décane.

### Stade A : 1-tert-butoxycarbonyl 10-(2,4-dichloro phénoxy acétyl) triaza 1,6,10-décane

A un mélange de 5,82 g de produit obtenu au stade B de l'exemple 9 avec 2,76 g de carbonate de potassium et 30 cm³ de terbutanol porté au reflux, on ajoute une solution de 4,5 g de 3-[(tert-butoxycarbonyl) amino] 1-bromopropane (préparation 3) dans 18 cm³ de terbutanol. Après 5 heures de reflux, on refroidit, filtre l'insoluble et évapore à sec le filtrat. On chromatographie le résidu (11,6 g) sur silice (éluant : méthanol). On obtient 3,44 g de produit attendu.

### Stade B : Dichlorhydrate de 1-(2,4-dichlorophénoxy acétyl) triaza 1,6,10-décane.

On agite au reflux pendant 4 heures un mélange de 3,44 g de produit obtenu au stade A, 100 cm³ d'éthanol et 13 ml d'une solution environ 2N d'éthanol chlorhydrique. On refroidit et essore, on obtient 2,4 g de produit attendu.
F = 220°C.

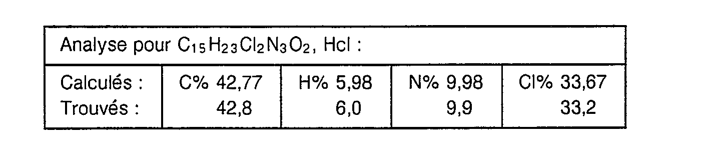

### EXEMPLE 11 : 1,10-bis-(2,4-dichlorophénoxy acétyl) 6-méthyl triaza 1,6,10-décane

A un mélange de 1,6 g de 6-méthyl triaza 1,6,10-décane (préparation ci-après), 20 cm³ de chloroforme et 2,02 g de triéthylamine, on ajoute, sans dépasser 30 C, 4,8 g de chlorure de l'acide 2,4-dichlorophénoxy acétique (préparation de l'exemple 9) en solution dans 15 cm³ de chloroforme. Après 3 heures d'agitation à 20°C, on évapore à sec sous pression réduite et on reprend le résidu avec 180 cm³ de tétrahydrofuranne, filtre l'insoluble et évapore le filtrat, à sec sous pression réduite on chromatographie le résidu (6,8 g) sur silice (éluant : chloroforme-méthanol (9-1 )). On obtient 4 g de produit attendu.

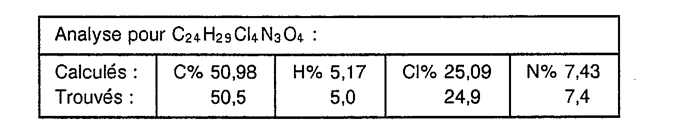

### Préparation de 6-méthyl triaza 1,6,10-décane utilisé au départ de l'exemple 11.

### Stade A : 3-méthyl 3-aza 1,6-dicyano hexane.

A un mélange agité à 20⁰ C de 42 g de méthyl amino propionitrile, 325 cm³ d'alcool terbutylique et 70 g de carbonate de potassium, on ajoute 75 g de 4-bromo-butyronitrile, on agite pendant 2 heures au reflux puis 16 heures à température ambiante. On filtre l'insoluble, concentre le filtrat et chromatographie le résidu (96 g) sur silice (éluant : chloroforme-acétone-cyclohexane (1-1-1)). On obtient 54 g de produit attendu.

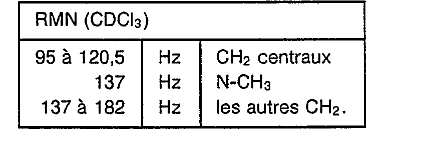

### Stade B : 6-méthyl triaza 1,6,10-décane.

A une solution de 3 g de soude pastille dans 55 cm³ d'éthanol 95, on ajoute 10 g de produit obtenu au stade A, 20 cm³ d'éthanol et 2 g de nickel de raney. On agite sous atmosphère d'hydrogène jusqu'à fin d'absorption complète. On filtre le catalyseur et concentre le filtrat à faible volume, ajoute 60 cm³ d'eau. On relargue avec de la lessive de soude, recueille l'huile ainsi séparée et extrait la phase aqueuse avec du chloroforme. Les extraits réunis avec l'huile sont concentrés à sec. On obtient 11 g de produit attendu.

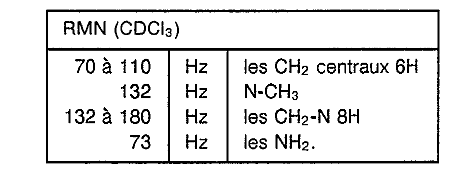

### EXEMPLE 12 : N-(1-naphtyl acétyl) 1,4-diamino butane et son chlorhydrate;

### Stade A : N-(tert-butoxycarbonyl) N'-(1-naphtyl acétyl) 1,4-diamino butane.

A une solution refroidie à +10° C de 15 g de N-(tert-butoxycarbonyl) 1,4-diamino butane (préparation 1), 100 cm³ de chloroforme et 14 cm³ de triéthylamine, on ajoute à +10°C une solution de 16,3 g de chlorure d'acide 1-naphtyl acétique dans 80 cm³ de chloroforme. Après 1 heure d'agitation à température ambiante, on concentre à sec sous pression réduite. On reprend le résidu à l'eau, essore et sèche sous pression réduite à 50°C. On obtient 27,8 g de produit attendu.

### Stade B : N-(1-naphtyl acétyl) 1,4-diamino butane et son chlorhydrate.

### a) Préparation du chlorhydrate :

A un mélange de 27,76 g du produit obtenu au stade A et 200 cm³ d'éthanol, on ajoute 86 cm³ d'éthanol-chlorhydrique -2N et l'on agite pendant 1 heure au reflux. On concentre à sec sous pression réduite. On obtient 23,4 g de chlorhydrate intermédiaire F - 130°C, isolé de l'acétate d'éthyle.

### b) Obtention de l'amine :

On reprend les 23,4 g de chlorhydrate intermédiaire avec 200 cm³ d'eau, on ajoute 80 cm³ de soude N et on agite 10 minutes à température ambiante. On extrait avec du chlorure de méthylène, sèche, filtre et évapore à sec sous pression réduite, on obtient 19,08 g de produit brut (F < 50 C) que l'on chromatographie sur silice (éluant : méthanol-ammoniaque (98-2)). Le produit obtenu est empâté dans l'acétate d'éthyle puis dans l'éther. On obtient 15,87 g de produit attendu.
F = 96° C.

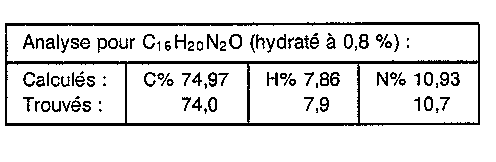

### Préparation de chlorure de l'acide 1-naphtyl acétique utilisé au départ de l'exemple 12.

On chauffe sous agitation, pendant 2 heures, au reflux, 15 g d'acide 1-naphtyl acétique et 90 cm³ de chlorure de thionyle. On concentre la solution sous pression réduite et on reprend le résidu par du toluène et concentre à sec sous pression réduite. On obtient 16,3 g de produit attendu utilisé tel quel au départ de l'exemple 12.

### EXEMPLE 13: Dichlorhydrate 1-(1-naphtyl acétyl) triaza 1,6,10-décane

### Stade A : 1-(1-naphtyl acétyl) 10-(tert-butoxycarbonyl) triaza 1,6,10-décane.

A une solution de 8,8 g de produit obtenu au stade B de l'exemple 12 dans 50 cm³ de terbutanol et 4,7 g de carbonate de potassium, on ajoute, au reflux, 8,1 g de 3-[(tert-butoxycarbonyl) amino] 1-bromopropane (préparation 3) en solution dans 40 cm³ de terbutanol. On agite 3 heures 30 au reflux et 16 heures à température ambiante. On filtre l'insoluble, concentre sous pression réduite. On chromatographie le résidu (17,21 g) sur silice (éluant : méthanol). On recueille 6,16 g de produit attendu.

### Stade B : Dichlorhydrate 1-(1-naphtyl acétyl) triaza 1,6,10-décane.

On agite 2 heures 30 au reflux, 5,9 g de produit obtenu au stade A, 60 cm³ d'éthanol et 26 cm³ d'éthanol chlorhydrique 2N. On essore, empâte acétate d'éthyle puis éther isopropylique. On obtient 4,78 g de produit attendu.
F = 218°C.

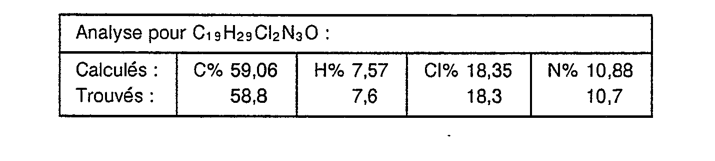

### EXEMPLE 14 : Chlorhydrate de N-(naphtoxy acétyl) 1,4-diamino butane

### Stade A : N'-(tert-butoxycarbonyl) N-(2-naphtyl acétyl) 1,4-diamino butane.

A une solution de 10,32 g de N-[(tert-butoxycarbonyl) 1,4-diamino butane (préparation 1) dans 110 cm³ de tétrahydrofuranne et 6,7 g de triéthylamine, on ajoute, entre 0°et +10°C, 11,16 g de chlorure d'acide naphtoxy acétique en solution dans 50 cm³ de tétrahydrofuranne. On agite 1 heure 30 à température ambiante. On filtre l'insoluble, évapore à sec et chromatographie le résidu (17,78 g) sur silice (éluant : hexane-acétate d'éthyle (1-1)). On obtient 10 g de produit attendu. F = 102°C.

### Stade B : Chlorhydrate de N-(naphtoxy acétyl) 1,4-diamino butane.

On agite au reflux pendant 1 heure 30, 7,66 g du produit obtenu au stade A, 80 cm³ d'éthanol et 14,7 cm³ d'éthanol chlorhydrique -2N. On refroidit lentement, essore et sèche. On obtient 4,56 g de produit attendu. F = 208 C.

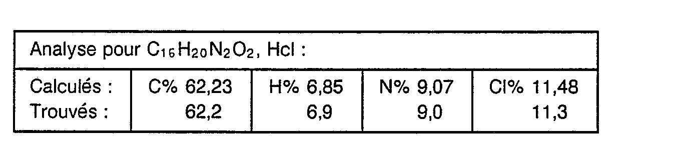

### Préparation du chlorure d'acide naphtoxy acétique, utilisé au départ de l'exemple 14.

On porte au reflux pendant 2 heures, 10 g d'acide naphtoxy acétique et 60 cm³ de chlorure de thionyle, on évapore à sec sous pression réduite et procède à plusieurs entraînements avec du benzène. On obtient 11,16 g de produit attendu, utilisé tel quel au départ de l'exemple 14.

### EXEMPLE 15 : Dichlorhydrate de 1-(2-naphtoxy acétyl) triaza 1,6,10-décane

### Stade A : 1-(2-naphtoxy acétyl) 10-(tert-butoxycarbonyl) triaza 1,6,10-décane.

A un mélange agité au reflux de 5 g de N-(2-naphtoxy acétyl) 1,4-diamino butane (préparation ci-après) 20 cm³ de terbutanol et 2,54 g de carbonate de potassium, on ajoute 4,37 g de 3-[(tert-butoxycarbonyl) amino] 1-bromopropane (préparation 3) et 20 cm³ de terbutanol. On agite 3 heures 30 au reflux et 16 heures à température ambiante. On filtre l'insoluble, évapore sous vide, chromatographie sur silice (éluant : méthanol à 0,5 % d'ammoniaque), on obtient 2,9 g de produit attendu.

### Stade B : Dichlorhydrate de 1-(2-naphtoxy acétyl) triaza 1,6,10-décane.

On agite 2 heures au reflux 2,823 g de produit obtenu au stade A, 14 cm³ d'éthanol et 8,5 cm³ d'éthanol chlorhydrique 2N. On essore sous argon et sèche sous pression réduite. On obtient 2 g de produit attendu. F > 260 C.

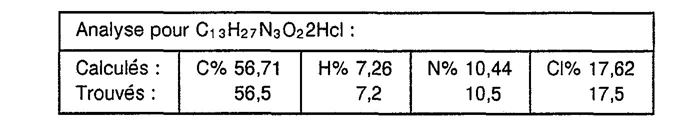

### Préparation de N-(2-naphtoxy acétyl) 1,4-diamino butane utilisé au départ de l'exemple 15.

On agite 4 heures au reflux un mélange de 10,32 g de produit obtenu à l'exemple 14, 200 cm³ d'éthanol et 3,37 g de bicarbonate de sodium. On évapore à sec et cristallisé dans l'éther isopropylique, on obtient 10 g de produit attendu.
F = 58° C.

### EXEMPLE 16 : N-2-allyl 3-trifluorométhylphénoxyacétyl) 1,4.diamino butane et son chlorhydrate.

### Stade A : N-(tert-butoxycarbonyl) N'-[(2-allyl 3-trifluorométhyl) phénoxyacétyl] diamino 1,4-butane.

On opère comme à l'exemple 1 en utilisant 4,99 g de N-(tert-butoxycarbonyl) 1,4-diaminobutane (préparation 1) et 7,40 g de chlorure d'acide (2-allyl 3-trifluorométhyl) phénoxy acétique, on obtient 8,83 g de produit attendu.

### Stade B : N-2-allyl 3-trifluorométhylphénoxyacétyl) 1,4-diamino butane et son chlorhydrate.

En opérant comme à l'exemple 1 à partir de 8,7 g de produit obtenu au stade A et 23 cm³ d'acide chlorhydrique environ 2N dans l'éthanol, on a obtenu 8,52 g de chlorhydrate que l'on alcalinise avec 23 cm³ de soude N. On obtient 1,37 g de produit attendu sous forme de base.

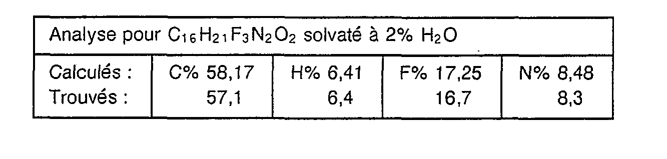

### Préparation du chlorure d'acide 2-allyl 3-trifluorométhylphénoxy utilisé au départ de l'exemple 16.

### Stade A : 3-trifluorométhylphénoxyallyle.

On ajoute 35,2 g de carbonate de potassium à 30 cm³ de 3-trifluorométhylphénol dans 50 cm³ de méthyléthylcétone. On chauffe 1 heure au reflux, laisse revenir à température ambiante, ajoute 26 cm³ de bromure d'allyle dans 10 cm³ de méthyéthylcétone, agite 1 heure, chauffe 1 heure et demie à reflux, laisse revenir à température ambiante et maintient 16 heures sous agitation. On extrait au chlorure de méthylène, sèche et concentre, on récupère 51,5 g de produit brut que l'on reprend dans l'eau, alcalinise à l'aide de soude N, extrait au chlorure de méthylène, sèche et concentre à sec. On obtient 48,21 g de produit attendu.

### Stade B : 2-allyl 5-trifluorométhylphénol et isomère 3-trifluorométhyle correspondant.

On chauffe 20 heures au reflux 29,7 g de produit obtenu au stade A, laisse revenir à température ambiante, chromatographie sur silice (éluant : n-hexane-acétate d'éthyle 9-1) et récupère 8,79 g de produit, rf 0,32 (isomère A 5CF₃) et 12,36 g de produit, rf 0,25 (isomère B 3CF₃).

### Stade C : 2- allyl 3-trifluorométhylphénoxy acétate d'éthyle.

On ajoute 5,8 g de carbonate de potassium à 6 g de produit 3-CF₃ obtenu au stade B dans 30 cm³ de méthyléthylcétone et chauffe 1 heure au reflux. On laisse revenir à température ambiante, ajoute 3,7 cm³ de bromoacétate d'éthyle, chauffe 1 heure au reflux, verse dans l'eau, extrait au chlorure de méthylène, sèche et concentre sous pression réduite. On reprend le produit brut obtenu dans l'eau, alcalinise à l'aide de soude N , extrait au chlorure de méthylène, sèche et évapore le solvant. On recueille 8,17 g de produit attendu.

### Stade D : Acide 2-allyl 3-trifluorométhylphénoxy acétique.

On agite 1 heure à température ambiante 8,1 g de produit obtenu au stade C dans 50 cm³ d'éthanol en présence de 14 cm³ de soude 2N. On ajoute alors 14 cm³ l'acide chlorhydrique 2N, agite 15 minutes et concentre. On obtient 10,19 g de produit brut que l'on reprend à l'eau. On extrait au chlorure de méthylène, sèche et évapore le solvant et obtient 7,26 g de produit attendu.

### Stade E : Chlorure de l'acide 2-allyl 3-trifluorométhylphénoxy acétique.

On chauffe 2 heures au reflux 7 g de produit obtenu au stade D dans 50 cm3 de chlorure de thionyle. On concentre, reprend le résidu au toluène, et élimine les solvants sous pression réduite. On obtient le produit brut utilisé tel quel au départ de l'exemple 16.

### EXEMPLE 17 : N-(2-allyl 5-trifluorométhylphénoxyacétyl) diamino 1,4-butane et son chlorhydrate.

### Stade A : N-tert-butoxycarbonyl N' -[2-allyl 5-trifluorométhylphénoxyacétyl] diamino 1,4-butane.

On opère comme à l'exemple 1 en utilisant 3,71 g de N-(tert-butoxycarbonyl) 1,4-diaminobutane préparation 1) et 5,5 g de chlorure d'acide (2-allyl 5-trifluorométhyl) phénoxy acétique. On obtient 8,36 g de produit attendu.

### Stade B : N-(2-allyl 5-trifluorométhylphénoxyacétyl) diamino 1,4-butane et son chlorhydrate.

On opère comme à l'exemple 1 en utilisant 8,20 g de produit obtenu au stade A et 21,1 cm³ d'acide chlorhydrique environ 2N dans l'éthanol et obtient 3,56 g de chlorhydrate que l'on alcalinise avec 9,7 cm³ de soude N. Après extraction au chlorure de méthylène, concentration de la phase organique et chromatographie du résidu sur silice (éluant : méthanol-ammoniaque 98-2), on obtient 2,17 g de produit attendu sous forme de base.

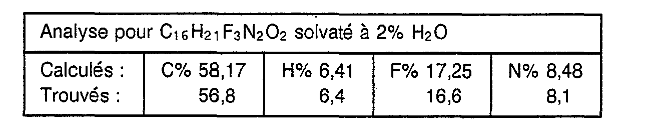

### Préparation du chlorure d'acide 2-allyl 5-trifluorométhylphénoxy acétique utilisé au départ de l'exemple 17.

### Stade A : 2-allyl 5-trifluorométhylphénoxy acétate d'éthyle.

On opère comme au stade C de l'exemple 16 en utilisant au départ 5 g de l'isomère A obtenu au stade B de l'exemple 16 et 3,1 cm³ de bromoacétate d'éthyle. On obtient 6,33 g de produit attendu.

### Stade B : Acide 2-allyl 5-trifluorométhylphénoxy acétique.

On opère comme au stade D de l'exemple 16 à partir de 6,25 g de produit obtenu au stade A, 10,9 cm³ de soude 2N dans 40 cm³ d'éthanol puis ajoute 10,9 cm³ d'acide chlorhydrique 2N. On récupère 5,28 g de produit attendu.

### Stade C : Chlorure de l'acide 2-allyl 5-trifluorométhylphénoxy acétique.

En opérant comme au stade E de l'exemple 16 à partir de 5,2 g de produit obtenu ci-dessus, 40 cm³ de chlorure de thionyle, on obtient 5,55 g de produit attendu utilisé tel quel au stade A de l'exemple 17.

### EXEMPLE 18 : Chlorhydrate de N-[6-(sec-butyl) 2,4-dichlorophénoxy] acétyl 5-(guanidine) aminopentane.

### 3tade A : N-[6-(sec-butyl) 2,4-dichlorophénoxy] acétyl 5-[(diterbutoxycarbonyl) guanidine) aminopentane.

On opère comme au stade A de l'exemple 7 en utilisant au départ 6 g de 5-(diterbutoxycarbonyl guanidine) aminopentane et 5,5 g de produit obtenu comme au stade A de l'exemple 6. On obtient après chromatographie sur silice (éluant : chlorure de méthylène), 6,52 g de produit attendu.

### 3tade B : Chlorhydrate de N-[6-(sec-butyl) 2,4-dichlorophénoxy] acétyl 5-(guanidine) aminopentane.

On ajoute 7 cm³ d'une solution éthanolique d'acide chlorhydrique (3,3N) à 6,52 g de produit obtenu au stade A en solution dans 65 cm³ d'éthanol et chauffe 24 heures au reflux. On refroidit à température ambiante, concentre à sec sous pression réduite, reprend le résidu dans l'eau et extrait à l'acétate d'éthyle. On obtient 3,55 g de produit attendu.

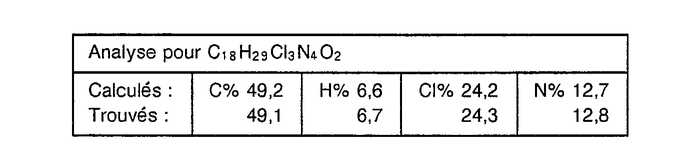

### Préparation du 5-(diterbutoxycarbonyl guanidine) aminopentane utilisé au départ de l'exemple 18.

### Stade A : 3-benzyloxycarbonyl 1,3-thiazolidine 2-thione.

A 10 g de mercaptothiazoline en solution dans 200 cm³ de chlorure de méthylène, on ajoute 12,9 cm³ de triéthylamine refroidie à 0/-5°C et ajoute goutte à goutte 12 cm³ de benzylchloroformiate en solution dans 60 cm³ de chlorure de méthylène. On agite 3 heures à température ambiante, verse dans une solution glacée d'acide chlorhydrique N, extrait la phase aqueuse au chlorure de méthylène, sèche la phase organique et la concentre à sec; on chromatographie le résidu sur silice (éluant : hexane-acétate d'éthyle 7-3) et obtient 17,9 g de produit attendu. F = 72 C.

### Stade B : N-benzyloxycarbonyl 1,5-diaminopentane.

On ajoute 17,84 g de produit obtenu au stade A en solution dans 100 cm³ de chlorure de méthylène à 16,4 g de 1,5- diaminopentane en solution dans 85 cm³ de chlorure de méthylène. On agite 3 heures, concentre à sec, chromatographie le résidu (éluant : méthanol-ammoniaque 99-1) et obtient 10,5 g de produit attndu.

### Stade C : N-benzyloxycarbonyl 5-[(diterbutoxycarbonyl) guanidine) aminopentane.

On chauffe 3 heures au reflux 10,5 g de produit du stade B et 14,2 g de N,N'bis-(terbutoxycarbonyl) S-méthylthioisourée dans 75 cm³ de tétrahydrofuranne et 5 cm³ d'eau. On refroidit le mélange, le verse sur une solution aqueuse de bicarbonate de sodium, extrait au chlorure de méthylène, lave la phase organique à l'eau, la sèche et concentre à sec sous pression réduite. On chromatographie le résidu sur silice (éluant : hexane-acétate d'éthyle 7-3) et obtient 10 g de produit attendu.

### Stade D : 5-[(diterbutoxycarbonyl) guanidine) aminopentane.

On hydrogène pendant 5 heures, 10 g de produit obtenu au stade C dans l'éthanol en présence de 1 g de charbon actif à 10% de palladium. On filtre le catalyseur, concentre à sec le filtrat et obtient 6,74 g de produit attendu.

### EXEMPLE 19 ; Chlorhydrate de N-[6-(secbutyl) 2,4-dichlorophénoxy] acétyl 3-guanidine aminopropane.

### Stade A : N-[6-(sec-butyl) 2,4-dichlorophénoxy] acétyl 3-[(diterbutoxycarbonyl) guanidine aminopropane.

On ajoute 1,1 g de bicarbonate de sodium dans 3,93 g de chlorhydrate de N-[6-(sec-butyl 2,4-dichlorophénoxy) acétyl] 1,3-diamino propane préparé dans J. Org. Chem. 52 (1987) 1700-1703, en solution dans 50 cm³ d'éthanol. On chauffe 4 heures et demie au reflux puis abandonne 12 heures à température ambiante. On concentre à sec, dilue à l'eau, extrait au chlorure de méthylène, concentre à sec la phase organique et obtient 3,47 g d'amine que l'on ajoute à 3,63 g de N,N'bis-(terbutoxycarbonyl) S-méthylthioi- sourée dans 20 cm³ de tétrahydrofuranne et 20 cm³ d'eau. On chauffe 3 heures et demie au reflux, refroidit à température ambiante, verse sur une solution aqueuse de bicarbonate de sodium à 10%. On extrait au chlorure de méthylène, réunit les phases organiques, sèche et concentre à sec sous pression réduite. Après chromatographie sur silice (éluant : hexane-acétate d'éthyle 75-25) on récupère 2,4 g de produit attendu.

### Stade B : Chlorhydrate de N-[6-(sec-butyl) 2,4-dichlorophénoxy] acétyl 3-guanidine aminopropane.

On opère comme au stade B de l'exemple 18 à partir de 2,4 g du produit obtenu ci-dessus et obtient 1,42 g du chlorhydrate attendu.

IR (CHCl₃) :
NH : 3418 - 3335 - 3165 cm⁻¹
C = O : 1669 cm⁻¹
C = C, C = N, CONH, amide II : 1620 - 1583 - 1565 - 1542 cm-'

### EXEMPLE 20 : Chlorhydrate de N-[6-(sec-butyl) 2,4-dichlorophénoxy] acétyl diamino 1,3-propane.

### Stade A : N'-[6-(sec-butyl) 2,4-dichlorophénoxy] acétyl N-terbutoxycarbonyl diamino 1,3-propane.

On opère comme au stade A de l'exemple 7 au départ de 1,68 g de l'amine obtenue comme indiqué à la préparation 2 et 3,4 g de produit obtenu comme au stade A de l'exemple 6. On obtient 3,7 g de produit attendu.

### Stade B : Chlorhydrate de N-[6-(sec-butyl) 2,4-dichlorophénoxy] acétyl diamino 1,3-propane.

On opère comme au Stade B de l'exemple 18 au départ de 3,69 g du produit préparé au stade A et 2,9 cm³ d'acide chlorhydrique 3,3 N. On obtient 3,05 g de produit attendu.

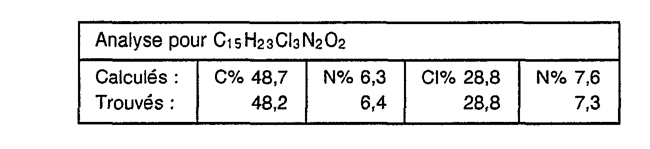

### EXEMPLE 21 : Chlorhydrate de N-[6-(sec-butyl) 2,4-dichlorophénoxy] acétyl diamino 1,5-pentane.

### Stade A : N'-[6-(sec-butyl) 2,4-dichlorophénoxy] acétyl N-terbutoxycarbonyl diamino 1,5-pentane.

On opère comme au stade A de l'exemple 7 au départ de 1,9 g de l'amine obtenue comme indiqué à la préparation 4 et 3,32 g de produit obtenu comme au stade A de l'exemple 6. On obtient 3,92 g de produit attendu.

### Stade B : Chlorhydrate de N-[6-(sec-butyl) 2,4-dichlorophénoxy] acétyl diamino 1,5-pentane.

On opère comme au Stade B de l'exemple 18 au départ de 3,88 g du produit préparé au stade A et 3 cm³ d'acide chlorhydrique 3,3 N. On obtient 2,88 g de produit attendu.

IR (CHCl₃) :
NH : 3425 cm-'
C = O : 1673 cm⁻¹
C=C, aromatique, CO amide Il : 1610 - 1584 - 1565 - 1538 cm⁻¹

### EXEMPLE 22 : Chlorhydrate de N-[6-(sec-butyl) 2,4-dichlorophénoxy] acétyl 4-guanidine aminobutane.

On opère comme au stade B de l'exemple 18 au départ de 4,49 g de produit préparé au stade A de l'exemple 18 et 5 cm³ d'acide chlorhydrique 3,3N. On obtient le chlorhydrate que l'on dilue dans le chlorure de méthylène, sèche et concentre à sec. Après recristallisation dans l'éther isopropylique, on obtient 2,4 g de produit attendu.

### EXEMPLE 23: Dichlorhydrate de (2-secbutyl 5-trifluorométhylphénoxyacétyl) triaza 1,6,10-décane.

### Stade A : N-(2-sec-butyl 5-trifluorométhylphénoxyacétyl) 10-terbutoxycarbonyl triaza 1,6,10-décane.

On refroidit à 0°/+5°C 3g d'acide [5-trifluorométhyl 2-(2-butyl) phénoxy] acétique dans 30 cm³ de chlorure de méthylène en présence de 1,44 g de 2-mercapto thiazoline et 0,30 g de 4-diméthylaminopyridine. On ajoute progressivement 2,47 g de dicyclohexylcarbodiimide en solution dans 20 cm³ de chlorure de méthylène, maintient l'agitation 1 heure à 0°C puis 16 heures à température ambiante. On filtre, concentre à sec le filtrat et obtient 5,46 g de résidu que l'on chromatographie sur silice (éluant : chlorure de méthylène) et obtient 2,70 g de produit que l'on dissout dans 30 cm³ de chlorure de méthylène et ajoute 1,8 g de produit préparé comme au stade D de la préparation de l'exemple 8 en solution dans 20 cm³ de chlorure de méthylène. On maintient 16 heures sous agitation à température ambiante, concentre à sec, et chromatographie le résidu sur silice (éluant : méthanol). On obtient 2,62 g de produit attendu.

### Stade B : Dichlorhydrate de (2-sec-butyl 5-trifluorométhylphénoxyacétyl) triaza 1,6,10-décane.

On chauffe 3 heures au reflux 1,48 g de produit obtenu au stade A dans 20 cm³ d'une solution méthanolique d'acide chlo rhydrique 2N, concentre sous pression réduite et recueille 1,21 g de produit attendu après séchage sous pression réduite.

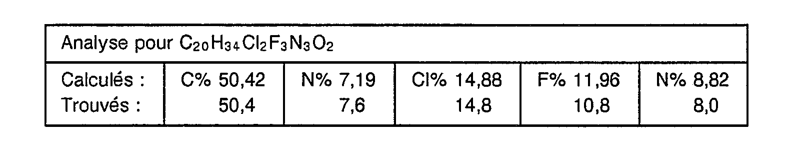

### Préparation de l'acide (5-trifluorométhyl 2-(1-méthylpropyl) phénoxy] acétique utilise au départ de l'exemple 23.

### Stade A : (3-trifluorométhylphénoxy) 2-butène.

On ajoute 18 g de carbonate de potassium à 15 cm³ de 3-trifluorométhylphénol et 25 cm³ de 2-butanone, agite, puis ajoute 15,1 cm^{3.}de 2-bromobutène dans 70 cm³ de 2-butanone et chauffe 1 heure et demie au reflux puis agite 72 heures à température ambiante. On ajoute alors 300 cm³ d'eau, extrait au chlorure de méthylène, sèche et concentre à sec et recueille 21,73 g de produit attendu.

### Stade B : 5-trifluorométhyl 2-(1-méthyl 2-propényl) phénol.

On chauffe 2 heures au reflux 123,3 g de produit préparé comme au stade A, Chromatographie sur silice (éluant : n-hexane-éther diisopropylique 9-1 puis 8-2) et récupère 16,95 g de produit attendu.

### Stade C : 5-trifluorométhyl 2-(1-méthylpropyl) phénol.

On hydrogène 4,59 g de produit du stade B dans 200 cm³ d'éthanol en présence de 0,50 g de charbon actif à 10% de palladium. On filtre, concentre le filtrat et obtient 4,05 g de produit attendu.

### Stade D : [5-trifluorométhyl 2-(1-méthylpropyl) phénoxy] acétate d'éthyle.

On ajoute 2,5 cm³ de bromoacétate d'éthyle à 4 g de produit obtenu au stade C dans 15 cm³ de diméthylsulfoxyde. On ajoute 4 g de carbonate de potassium puis chauffe à 80°C pendant 2 heures et demie. On verse le milieu réactionnel dans l'eau glacée, extrait au chlorure de méthylène, sèche et concentre à sec. On obtient 5,10 g de produit attendu.

### Stade E : Acide [5-trifluorométhyl 2-(1-méthylpropyl)] acétique.

On agite 45 minutes à température ambiante 3 g de produit obtenu au stade D et 8 cm³ de soude 2N dans 10 cm³ d'éthanol. On concentre puis dilue à l'eau, extrait au chlorure de méthylène, acidifie à pH = 1 puis extrait à l'acétate d'éthyle, lave à l'eau, sèche et élimine les solvants sous pression réduite. On obtient après lavage à l'hexane 1,24 g de produit attendu. F = 96⁰ C.

### EXEMPLE 24: N-(3-méthoxy 4-hydroxyphénoxyacétyl) diamino 1,4-butane et son chlorhydrate.

### Stade A : N-(3-méthoxy 4-benzyloxyphénoxyacétyl) N-terbutoxycarbonyl diamino 1,4-butane.

On opère comme au stade A de l'exemple 23 en utilisant au départ 2,8 g d'acide 4-benzyloxyméthoxy- phénoxy acétique, 115 mg de diméthylaminopyridine, 2 g de dicyclohexylcarbodilmide et 1,8 g de N-terbutoxycarbonyl diamino 1,4-butane. On obtient après chromatographie sur silice (éluant : chloroforme-acétone-cyclohexane 1-1-2) 2,4 g de produit attendu.

### Stade B : N-(3-méthoxy 4-hydroxyphénoxyacétyl) N'-terbutoxycarbonyl diamino 1,4-butane.

On hydrogène 2,4 g de produit préparé au stade A dans 100 cm³ d'éthanol avec 50 cm3 de cyclohexène en présence de 2,4 g de charbon actif à 10% de palladium. On chauffe au reflux 3 heures, refroidit, filtre et concentre à sec le filtrat. On obtient 1,96 g de produit attendu.

### Stade C : N-(3-méthoxy 4-hydroxyphénoxyacétyl) diamino 1,4-butane et son chlorhydrate.

On opère comme au stade B de l'exemple 1 à partir de 1,96 g de produit obtenu ci-dessus. On obtient 1,56 g de chlorhydrate puis 1,28 g de la base attendue après chromatographie sur silice (éluant : méthanol-ammoniaque 98-2).

F = 100°C.

IR (CHCl₃) :
OH-NH : 3303 cm-'
C = O : 1652 cm⁻¹
NH₂, aromatique, amide II: 1612 - 1574 - 1545 - 1491 cm-¹

### Préparation de l'acide 4-benryloxy 3-méthoxyphénoxy acétique utilisé au départ de l'exemple 24.

### Stade A : 4-benzyloxy 3-méthoxyphénol.

On dissout 24,2 g de 4-benzyloxy 3-méthoxy benzaldéhyde (Beilst. 8 II. 283) dans 100 cm³ d'acide acétique, ajoute un mélange de 25 cm³ d'acide peracétique et 50 cm³ d'acide acétique sans dépasser 45 C et agite 18 heures à +40°C. On concentre à sec, chromatographie le résidu sur silice (éluant : hexane-acétate d'éthyle 8-2) et obtient 9,1 g de produit attendu. F = 84°C.

### Stade B : 4-benzyloxy 3-méthoxyphénoxy acétate d'éthyle.

On opère comme au stade C de l'exemple 16 à partir de 2,76 g de produit préparé au stade A, 1,82 g de carbonate de potassium, 15 cm³ de méthyléthylcétone et 1,6 cm³ de bromoacétate d'éthyle. L'extraction est effectuée à l'aide de méthyléthylcétone. La phase organique est concentrée à sec. On obtient 3,9 g de produit brut que l'on chromatographie sur silice (éluant : hexane-acétate d'éthyle 8-2). On obtient 3,6 g de produit attendu. F = 58°C.

### Stade C : Acide 4-benzyloxy 3-méthoxyphénoxy acétique.

On opère comme au stade D de l'exemple 16 au départ de 3,2 g de produit obtenu ci-dessus, 5 cm³ de soude 2N puis 5 cm³ d'acide chlorhydrique et obtient 2,85 g de produit attendu.

### EXEMPLE 25 : N-(2,4-dichlorophénoxyacétyl) N'-[1-(3-indolacétyl)] diamino 1,4-butane.

On opère comme à l'exemple 23 stade A au départ de 2 g d'acide 3-indolyl acétique, 0,135 g de diméthylaminopyridine, 2,35 g de dicyclohexylcarbodiimide et 3,32 g de N-(2,4-dichlorophénoxyacétyl) diamino 1,4-butane obtenu à partir du produit préparé à l'exemple 9. On obtient, après chromatographie sur silice (éluant : hexane-acétate d'éthyle 6-4) puis méthanol, 3,4 g de produit attendu. F = 128°C.

### EXEMPLE 26 : Chlorhydrate de N-(7-hydroxy coumarinyl 4-acétyl) diamino 1,4-butane.

### Stade A : N-terbutoxycarbonyl N'-(7-hydroxy coumarinyl 4-acétyl) diamino 1,4-butane.

On ajoute 8,9 g d'acide 7-hydroxy coumarin 4-acétique dans 135 cm³ de tétrahydrofuranne à une suspension comprenant 12,64 g d'iodure de 2-chloro N-méthyl pyridinium dans 135 cm³ de tétrahydrofuranne. On ajoute ensuite 7,52 g de N-terbutoxycarbonyl diamino 1,4-butane obtenu comme à la préparation 1 dans 100 cm³ de tétrahydrofuranne puis 13,5 cm³ de triéthylamine et chauffe 6 heures au reflux puis maintient sous agitation pendant 16 heures à température ambiante. On filtre, concentre le filtrat, reprend le résidu dans l'eau, sèche les cristaux que l'on purifie par chromatographie sur silice (éluant : acétate d'éthyle-n-hexane 8-2). On obtient 4,46 g de produit attendu. F = 171 C.

### Stade B : Chlorhydrate de N-(7-hydroxy coumarinyl 4-acétyl) diamino 1,4-butane.

On opère comme à l'exemple 8 stade B à partir de 4,32 g de produit obtenu au stade A et 7,5 cm³ d'acide chlorhydrique 2,2N. On obtient 2,67 g de produit attendu après recristallisation dans le méthanol. F = 220 C.

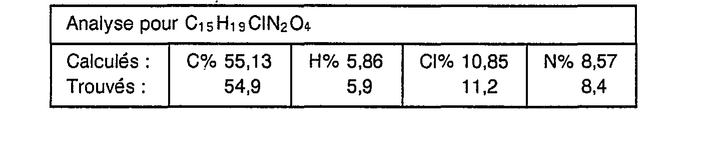

### Préparation de l'acide 7-hydroxy coumarin 4-acétique utilisé au départ de l'exemple 26.

On agite 1 heure à température ambiante 84 g d'acide citrique et 112 cm³ d'acide sulfurique concentré, chauffe progressivement à 70 C, maintient 35 minutes, refroidit à 0°C et ajoute 34,56 g de 1,3-benzène diol et 44,8 cm³ d'acide sulfurique concentré. On maintient 16 heures à 0°C, verse sur de la glace, essore le produit cristallisé, sèche sous pression réduite. On obtient après recristallisation dans l'eau 22,77 g de produit attendu. F = 210°C.

### EXEMPLE 27 : Chlorhydrate de N-[(2-méthyl 6-indolyloxy) acétyl] 1,4-diamino butane.

### Stade A : N-(tert-butoxycarbonyl) N'-[(2-méthyl 6-indolyloxy) acétyl] 1,4-diamino butane

On ajoute progressivement 1,07 g d'hydrure de sodium en suspension dans 50% d'huile dans une solution renfermant 3,28 g de 6-hydroxy 2-méthyl indole dans 50 cm³ de diméthylformamide. On agite la suspension ainsi ontenue pendant 1 heure. On introduit lentement une solution renfermant 6,5 g de N -(tert-butoxycarbonyl) N-(chloroacétyl) 1,4-diamino butane obtenu comme indiqué dans la préparation de l'exemple 3 et 60 cm³ de diméthylformamide. On agite le milieu réactionnel à 90°C pendant 5 heures, puis 16 heures à la température ambiante. On amène la suspension obtenue à sec. On obtient 13,08 g d'un produit que l'on chromatographie sur silice en éluant par le mélange hexane-acétate d'éthyle (30-70). On obtient 4,98 g du produit recherché fondant à 132° C.

### Stade B : Chlorhydrate de N-[(2-méthyl 6-indolyloxy) acétyl] 1,4-diamino butane.

On ajoute 9,9 cm³ d'une solution chlorhydrique 2N dans l'éthanol dans une suspension renfermant 4,96 g du composé obtenu au stade précédent dans 50 cm³ d'éthanol. On maintient le mélange réactionnel sous agitation pendant 1 heure au reflux. On amène la solution obtenue à sec. On obtient 5,70 g de produit brut que l'on reprend dans 20 cm³ d'acétonitrile. On ajoute 3 cm³ d'oxyde de propylène. On maintient le mélange réactionnel sous agitation pendant 30 minutes. On concentre le mélange réactionnel obtenu. On empâte le produit obtenu dans l'éther isopropylique, l'essore, le sèche. On obtient alors 3,88 g du produit recherché fondant à 129 C.

### EXEMPLE 28 : N-(3-trifluorométhylphénoxyacétyl) N-[1-(2-trifluoromethyl 3-indolacétyl)] diamino 1,4-butane

On opère comme au stade A de l'exemple 26 au départ de 6,01 g d'iodure de 2-chloro N-méthyl pyridinium, 4,77 g d'acide 3-(2-trifluorométhyl indolyl) acétique, 5,51 g de N-(3-trifluorométhylphénoxyacétyl) 1,4-diamino butane obtenu à partir du chlorhydrate préparé à l'exemple 4 et 6,55 cm³ de triéthylamine. Après chromatographie sur silice (éluant : acétate d'éthyle), on obtient 4,85 g de produit attendu.

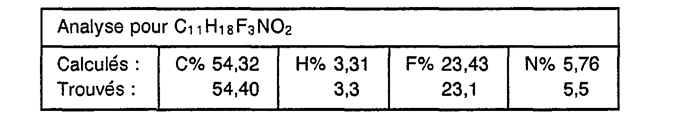

### Préparation de l'acide 3-(2-trifluoromethylindolyl) acétique utilisé au départ de l'exemple 28.

### Stade A : 5,5,5-trifluoro 4-oxo 3-éthoxycarbonyl pentanoate d'éthyle.

On mélange 200 g de succinate de diéthyle et 82 g de trifluoroacétate d'éthyle et ajoute en 45 minutes 13,2 g de sodium et 200 cm³ d'éther. On chauffe 18 heures à 80 °C, verse le milieu réactionnel sur 200 cm³ d'acide sulfurique 10N glacé. On décante, sèche, concentre et obtient le produit attendu que l'on utilise tel quel pour le stade suivant.

### Stade B : 5,5,5-trifluoro 4-oxo pentanoate d'éthyle.

On chauffe à 180° C pendant 3 heures 45 g de produit préparé au stade A, 10,3 g d'acide borique et 1 g d'acide paratoluènesulfonique. On verse sur de la glace, extrait à l'acétate d'éthyle, décante, sèche et concentre sous pression réduite. On distille le résidu et obtient 10,8 g de produit attendu.

### Stade C : 3-(2-trifluorométhylindolyl) acétate d'éthyle.

On ajoute une solution de 5,9 g de phénylhydrazine dans 10 cm³ d'éthanol à 10,8 g de produit préparé au stade B en solution dans 15 cm³ d'éthanol, puis 2,74 cm³ d'acide chlorhydrique 2N en solution dans l'éthanol. On agite 3 heures à 20°C, concentre sous pression réduite et obtient 17,6 g d'intermédiaire que l'on reprend dans 50 cm³ d'éthanol, refroidit à +4° C et traite par un courant d'acide chlorhydrique gazeux. On chauffe 24 heures à ébullition, filtre, concentre le filtrat, chromatographie le résidu sur silice (éluant : cyclohexane-éther isopropylique 7-3) et obtient 6,9 g de produit attendu. F = 86⁰ C.

### Stade D : Acide 3-(2-trifluorométhylindolyl) acétique.

On ajoute en 3 fois 2,15 g de bicarbonate de soude dans une solution de 3,08 g de produit obtenu ci-dessus dans 20 cm³ de méthanol et 10 cm³ d'eau et agite 24 heures. On concentre la solution, reprend le résidu par 20 cm³ d'eau, acidifie avec de l'acide chlorhydrique, extrait à l'acétate d'éthyle, sèche et concentre à sec. On chromatographie le résidu sur silice (éluant : hexane-acétate d'éthyle-acide acétique 78-20-2) et obtient 2,6 g de produit attendu. F = 146°C.

### EXEMPLE 29 ; 1,10-(3-indolacétyl) 1,6,10-triaza décane

En opérant comme indiqué précédemment à partir de l'acide et de l'amine appropriés, on a obtenu le produit attendu.

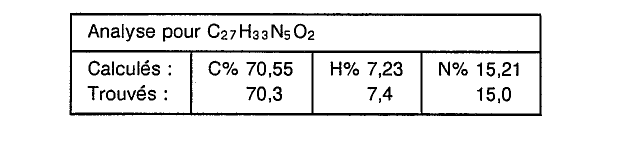

### Préparation 1 : N-(tert-butoxycarbonyl) 1,4-diamino butane.

A une solution de 320 g de 1,4-diamino butane, dans 2800 cm³ de chlorure de méthylène, refroidie à +5°C, on ajoute en 1 heure à 0°/+5°C, 400 g d'oxyde de di-tert-butoxycarbonyle en solution dans 2000 cm³ de chlorure de méthylène. On agite pendant 1 heure à 20⁰ C et filtre l'insoluble. On évapore le filtrat à sec sous pression réduite et on chromatographie sur silice l'huile obtenue (363,1 g) (éluant : flugène (113)-methanol (9-1) puis méthanol, puis méthanol-ammoniaque (96-4)). On obtient 203,6 g de produit attendu.

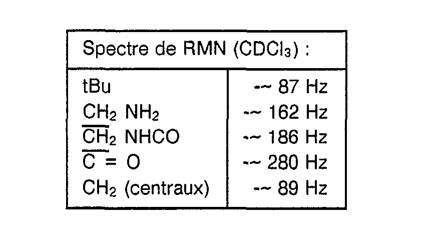

### Préparation 2 : N-(tert-butoxycarbonyl) 1,3-diamino propane.

A une solution de 36 cm³ de 1,3-diamino propane, dans 160 cm³ de chlorure de méthylène on ajoute à +5°C, sans dépasser +10°C, une solution de 46 g de carbonate de di-tert-butyle dans 120 cm³ de chlorure de méthylène, on agite 16 heures à 20 C, filtre l'insoluble et évapore le filtrat à sec sous vide. On obtient 63 g de résidu, chromatographie sur silice (éluant : méthanol à 2 % d'ammoniaque). On obtient 23 g de produit attendu. F < 60°C.

### Préparation 3 : 3-[(tert-butoxycarbonyl) amino] 1-bromopropane.

### Stade A : 3-[(tert-butoxycarbonyl) amino] 1-propanol.

A une solution de 7,5 g de 3-amino 2-propanol dans 75 cm³ d'eau et 75 cm³ de tétrahydrofuranne, on ajoute à température ambiante, 21,8 g de di-tert-butyl dicarbonate en solution dans 50 cm³ de tétrahydrofuranne. Après 2 heures d'agitation à 20 C, on évapore à sec sous pression réduite, on reprend le résidu huileux avec 150 cm³ d'acétate d'éthyle, lave la solution avec deux fois 30 cm³ d'acide acétique 0,5 M, puis à l'eau, on évapore à sec sous vide. On obtient 15,4 g de produit attendu.

### Stade B : 3-[(tert-butoxycarbonyl) amino] 1-bromopropane.

A une solution de 9 g de produit obtenu au stade A dans 120 cm³ de tétrahydrofuranne et 19,7 g de triphényl phosphine, on ajoute lentement, en maintenant la température entre 20 et 25 C, 25 g de tétrabromure de carbone en solution dans 50 cm³ d'acétonitrile. On agite pendant 15 heures, on filtre l'insoluble et concentre le filtrat à sec, sous pression réduite. On chromatographie le résidu (45 g) sur silice (éluant : hexane-acétate d'éthyle (8-2)). On obtient 9,4 g de produit attendu.
F = 40°C.

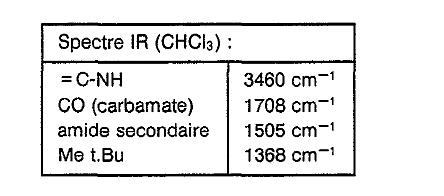

### Préparation 4 : N-tert-butoxycarbonyl diamino 1,5-pentane.

On refroidit à +5°C 54 cm³ de 1,5-diamino pentane dans 200 cm³ de chlorure de méthylène puis ajoute 50 g de diter-butyl dicarbonate en solution dans du chlorure de méthylène. On agite 15 minutes à 0°C, laisse revenir à température ambiante et maintient sous agitation pendant 16 heures. On filtre, concentre le filtrat à sec et obtient 38 g de produit brut que l'on chromatographie sur silice (éluant : méthanol-ammoniaque 98-2). On recueille 18,31 g de produit attendu.

### Activité biologique fongicide

### Description des tests.

### a) Inhibition de la germination de spores;

L'activité inhibitrice de la germination est mesurée sur les spores d'Alternaria solani et de Fusarium roseum. Les produits sont dissous dans le "Napsol PM1 ". Chaque solution est diluée avec 8 ml de "PDA" chaud (0,9 % de "Napsol PM1" dans la solution finale) dans une boite de Petri (50 mm de diamètre). Chaque produit est testé à la concentration de : 100 ou 1000 ppm. Quand le milieu est gélifié, la suspension de spores est déposée à la surface, à raison de 200 microlitres par boite, la suspension renfermant 100.000 spores par ml. La lecture de la germination s'effectue 18 heures après l'ensemencement. Les valeurs rapportées dans le tableau ci-dessous donnent le pourcentage d'inhibition de spores germées dans deux boîtes.
"Napsol PM1" : éther méthylique du monopropylène glycol.
"PDA" : Agar glucosé à la pomme de terre.

### b) Essais Botrytis cinerea sur vigne.

Les jeunes plants de vigne issus de boutures (variété Grenache N, clone 70) sont cultivés en serre (température de jour : 30 C, température de nuit : 25 C) sur mélange terre/terreau/sable, (1/3-1/3-1/3). Deux jours avant l'essai, les plants sont transportés en chambre de culture (mêmes conditions de température, humidité : 60 % le jour, 80 % la nuit). Le produit est dissous dans la "matrice A" à une concentration de 500 ppm juste avant l'utilisation. Le traitement se fait par pulvérisation de la solution sur les feuilles jusqu'à rétention maximale. Les spores de Botrytis cinerea sont mises en suspension dans du jus de carottes dilué, à raison de 50.000 spores par ml. La contamination s'effectue par dépôt de la suspension de spores sous forme de gouttes (20 microlitres) à la surface abaxiale des feuilles. Dans l'essai préventif, le traitement est effectué un jour avant la contamination. Dans l'essai curatif, la contamination est effectuée deux jours avant le traitement. Les plants sont ensuite maintenus dans la chambre de culture, aux mêmes conditions que précédemment. La lecture est faite neuf jours après la contamination, par mesure des surfaces nécrosées. L'efficacité du produit est calculée par rapport à un témoin non traité.

### c) Essais Plasmopara viticola.

Le matériel végétal utilisé est le même que celui utilisé dans l'essai B, et cultivé dans les mêmes conditions. Le traitement s'effectue de la même façon également. La contamination est faite avec une suspension de zoosporanges de Plasmopara viticola prélevés immédiatement avant l'essai (50.000 zoosporanges par ml). Les gouttes de suspension (20 microlitres) sont déposées à la face abaxiale des feuilles. Les plants sont ensuite maintenus pendant 24 heures dans une atmosphère saturée en humidité, puis ramenés à l'humidité de la chambre de culture (60 % le jour, 80 % la nuit). La lecture s'effectue dix jours après la contamination, par mesure du développement des îlots de conidiophores à la surface abaxiale des feuilles. L'efficacité du produit est calculée par rapport à un témoin non traité.

### d) Essais Erysiphe graminis hordei.

Les grains d'orge (variété Pression) sont mis à germer dans le mélange terre-terreau-sable (1/3-1/3-1/3) et cultivés en serre. Les produits sont dissous dans la "matrice A juste avant l'essai, à une concentration de 500 ppm. Le traitement s'effectue par pulvérisation de la solution de produit sur les plants d'orge âgés de dix jours, jusqu'à rétention maximale. La contamination par les conidies d'Erysiphe graminis hordei est faite trois jours après le traitement. Les plants sont maintenus en salle climatisée (température de jour : 23 C, température de nuit : 18°C). Sept jours après la contamination, on mesure l'étendue du feutrage conidien sur la première et la deuxième feuille de chaque plant. L'efficacité du produit est calculée par rapport à un témoin non traité.

### e) Essais Puccinia recondita tritici.

Les grains de blé (variété Festival) sont mis à germer dans le mélange terre-terreau-sable (1/3-1/3-1/3). Les plants sont cultivés en serre. Les produits sont dissous dans la "matrice A" juste avant l'essai, à une concentration de 500 ppm. Le traitement s'effectue par pulvérisation de la solution de produit sur les plants de blé âgés de neuf jours, jusqu'à rétention maximale. La contamination par les urédospores de Puccinia recondita tritici est faite le lendemain du traitement. Les plants sont maintenus en salle climatisée (température de jour : 22 C, température de nuit : 18°C). Sept jours après la contamination, on mesure la densité des sores sur les deux premières feuilles de chaque plant. L'efficacité du produit est calculée par rapport à un témoin non traité.

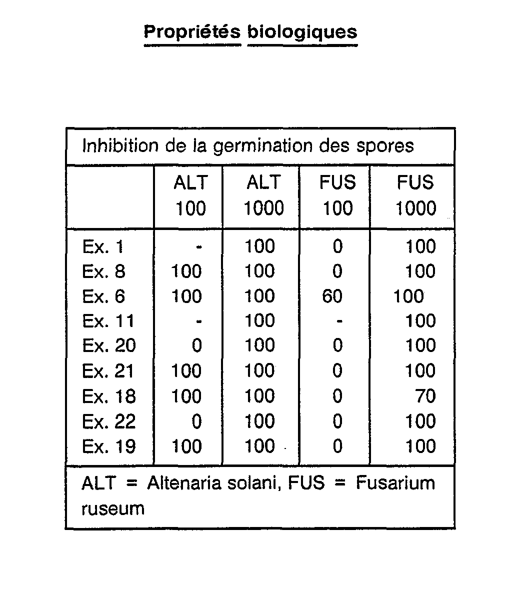

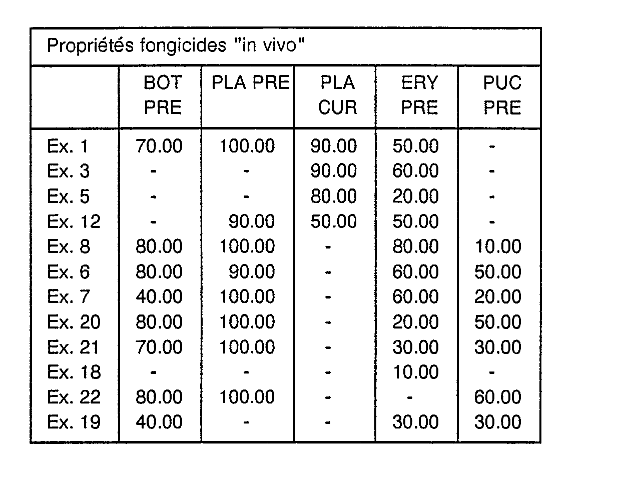

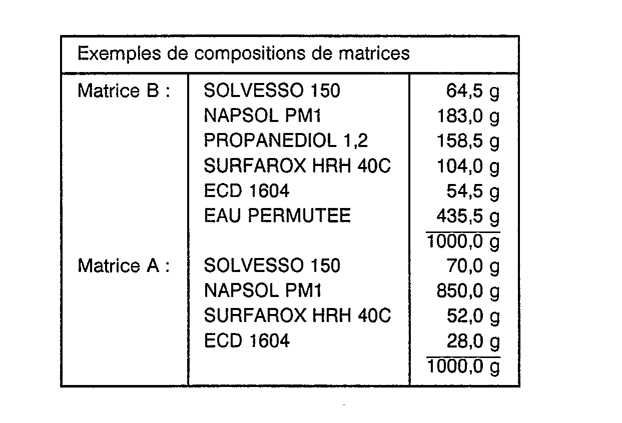

## Revendications

1) Les composés de formule générale (I) : 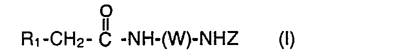 dans laquelle,
R, représente:
- soit un radical ArO- dans lequel Ar représente un radical aryle renfermant jusqu'à 14 atomes de carbone éventuellement substitué ou un radical hétéroaryle éventuellement substitué ou un radical hétérocyclique éventuellement substitué,
- soit un radical aryle ou polyaryle condensé ou non ou un radical hétérocyclique éventuellement substitué. W représente :
- soit un radical (CH₂)ₙ₁ dans lequel ni représente un nombre entier pouvant varier de 2 à 6,
- soit un radical (CH₂)n₂ NY(CH₂)n₃ dans lequel n₂ et n₃ identiques ou différents représentent un nombre entier pouvant varier de 2 à 6 et Y représente un atome d'hydrogène ou un radical alkyle renfermant jusqu'à 12 atomes de carbone ou un radical aryle renfermant jusqu'à 14 atomes de carbone éventuellement substitué ou un radical arylalkyle renfermant jusqu'à 18 atomes de carbone éventuellement substitué, ou un radical C0₂-alkyle dans lequel le radical alkyle renferme jusqu'à 12 atomes de carbone, ou un radical COR₂ dans lequel le radical R₂ représente un radical alkyle, alcényle ou alcynyle renfermant jusqu'à 12 atomes de carbone ou un radical aryle renfermant jusqu'à 14 atomes de carbone ou un radical arylalkyle renfermant jusqu'à 18 atomes de carbone éventuellement substitué ou un radical hétérocyclique éventuellement substitué et
Z représente :
soit un atome d'hydrogène,
- soit un radical 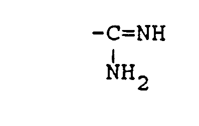
- soit un radical 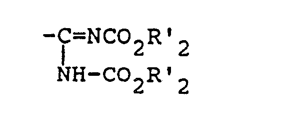 dans lequel R'₂ représente un radical alkyle renfermant jusqu'à 12 atomes de carbone, ou un radical arylalkyle renfermant jusqu'à 18 atomes de carbone,
- soit un radical C0₂-alkyle dans lequel le radical alkyle renferme jusqu'à 12 atomes de carbone,
- soit un radical C0₂-arylalkyle dans lequel le radical arylalkyle renferme jusqu'à 18 atomes de carbone,
- soit un radical COR"₂ dans lequel R"₂ peut prendre l'une des valeurs indiquées ci-dessus pour R₂,
- soit un radical -CH₂R₃ dans lequel R₃ représente un radical aryle éventuellement substitué, hétéroaryle éventuellement substitué, hétérocyclique éventuellement substitué ou peut prendre les valeurs indiquées ci-dessus pour Ri,
- soit un radical -COCH₂R₃ dans lequel R₃ peut prendre l'une des valeurs indiquées ci-dessus pour R₃, à la condition que Z ne représente pas un atome d'hydrogène, si W représente un radical (CH₂)₃ et R₁ représente un radical 2,4-dichlorophénoxy ainsi que les sels d'addition avec les acides organiques ou minéraux des composés de formule (I).

2) Les composés de formule (I) tels que définis à la revendication 1, répondant à la formule (I') : 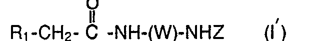 dans laquelle,
Ri représente:
- soit un radical ArO- dans lequel Ar représente un radical aryle renfermant jusqu'à 14 atomes de carbone éventuellement substitué ou un radical hétéroaryle éventuellement substitué, ou un radical hétérocyclique éventuellement substitué,
- soit un radical aryle, ou polyaryle condensé ou non ou un radical hétérocyclique éventuellement substitué. W représente :
- soit un radical (CH₂)ₙ₁ dans lequel ni représente un nombre entier pouvant varier de 3 à 6,
- soit un radical (CH₂)n₂ NY(CH₂)n₃ dans lequel n₂ et n₃ identiques ou différents représentent un nombre entier pouvant varier de 2 à 6 et Y représente un atome d'hydrogène ou un radical alkyle renfermant jusqu'à 12 atomes de carbone ou un radical aryle renfermant jusqu'à 14 atomes de carbone éventuellement substitué ou un radical arylalkyle renfermant jusqu'à 18 atomes de carbone éventuellement substitué, ou un radical C0₂ alkyle dans lequel le radical alkyle renferme jusqu'à R₂ atomes de carbone, ou un radical COR₂ dans lequel le radical R₂ représente un radical alkyle, alcényle ou alcynyle renfermant jusqu'à 12 atomes de carbone ou un radical aryle renfermant jusqu'à 14 atomes de carbone ou un radical arylalkyle renfermant jusqu'à 18 atomes de carbone éventuellement substitué ou un radical hétérocyclique éventuellement substitué et
Z représente :
- soit un atome d'hydrogène,
- soit un radical 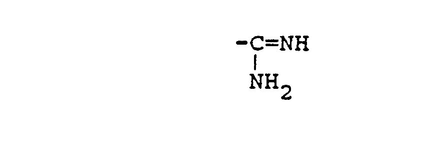
- soit un radical 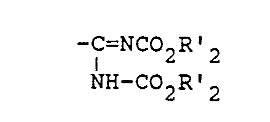 dans lequel R'₂ représente un radical alkyle renfermant jusqu'à 12 atomes de carbone, ou un radical arylalkyle renfermant jusqu'à 18 atomes de carbone,
- soit un radical C0₂ alkyle dans lequel le radical alkyle renferme jusqu'à 12 atomes de carbone,
- soit un radical C0₂ arylalkyle dans lequel le radical arylalkyle renferme jusqu'à 18 atomes de carbone,
- soit un radical -CH₂R₃ dans lequel R₃ représente un radical aryle éventuellement substitué, hétéroaryle éventuellement substitué, hétérocyclique éventuellement substitué ou peut prendre les valeurs indiquées ci-dessus pour Ri,
- soit un radical -COCH₂R₃ dans lequel R'₃ peut prendre l'une des valeurs indiquées ci-dessus pour R₃, à la condition que R, ne représente ni un radical 2,5-dihydroxyphényle, ni un radical 2,5-diméthoxyphényle et que Z ne représente pas un atome d'hydrogène, si W représente un radical (CH₂)₃ et R₁ représente un radical 2,4-dichlorophénoxy ainsi que les sels d'addition avec les acides organiques ou minéraux des composés de formule (I').

3) Les composés de formule (I) ou (I') tels que définis à la revendication 1 ou 2, dans lesquels les radicaux aryle, hétéroaryle, arylalkyle et hétérocyclique sont éventuellement substitués par un ou plusieurs substituants choisis dans le groupe constitué par les radicaux hydroxyle, les atomes d'halogène, les radicaux alkyle, alcényle et alcynyle renfermant jusqu'à 14 atomes de carbone, les radicaux CF₃, les radicaux OR"₃ dans lequel R"₃ représente un radical alkyle renfermant jusqu'à 12 atomes de carbone ou un radical arylalkyle renfermant jusqu'à 18 atomes de carbone, les radicaux COalkyle, Si(alkyle)₃, SO₂alkyle dans lesquels le radical alkyle renferme jusqu'à 12 atomes de carbone, et les radicaux SO₂aryle dans lesquels le radical aryle renferme jusqu'à 14 atomes de carbone, les différents radicaux pouvant former entre eux avec les atomes auxquels ils sont liés des cycles 0-CH₂-0, ainsi que leurs sels d'addition avec les acides organiques ou minéraux.

4) Les composés de formule (I) tels que définis à la revendication 1, 2 ou 3 dans lesquels W représente un radical -(CH₂)₃-(CH₂)₄- ou (CH₂)₅- ainsi que leurs sels d'addition avec les acides organiques ou minéraux.

5) Les composés de formule (I) tels que définis à la revendication 1, 2 ou 3 dans lesquels W représente un radical (CH₂)ₙ₄NH(CH₂)ₙ₅ dans lequel n₄ et n₅ identiques ou différents représentent les nombres 3, 4 ou 5 ainsi que leurs sels d'addition avec les acides organiques ou minéraux.

6) Les composés de formule (I) tels que définis à l'une quelconque des revendications 1 à 5 dans lesquels Z représente un atome d'hydrogène, ou un radical CO₂alkyle dans lequel le radical alkyle renferme jusqu'à 12 atomes de carbone ainsi que leurs sels d'addition avec les acides organiques ou minéraux.

7) Les composés de formule (I) tels que définis à l'une quelconque des revendications 1 à 6 dans lesquels Z représente un radical 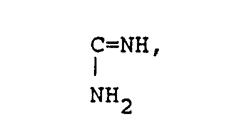 ou un radical 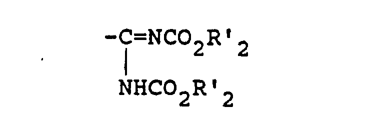 R'₂ gardant la même signification que dans la revendication 1, ainsi que leurs sels d'addition avec les acides organiques ou minéraux.

8) Les composés de formule (I) tels que définis à l'une quel conque des revendications 1 à 7 dans lesquels Rᵢ représente un radical ArO- dans lequel Ar représente un radical aryle éventuellement substitué ainsi que leurs sels d'addition avec les acides minéraux ou organiques.

9) Les composés de formule (I) tels que définis à la revendication 8 dans lesquels Ar représente le radical : 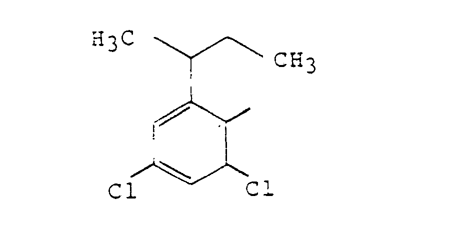 ainsi que leurs sels d'addition avec les acides organiques ou minéraux.

10) Les composés de formule (I) tels que définis à l'une quelconque des revendications 1 à 7 dans lesquels Rᵢ représente un radical : 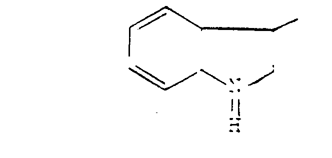 ainsi que leurs sels d'addition avec les acides organiques ou minéraux.

11) Les composés de formule (I) tels que définis à l'une quelconque des revendications 1 à 7 dans lesquels R₁ représente un radical : 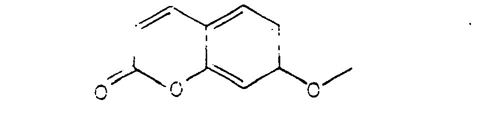 ainsi que leurs sels d'addition avec les acides organiques ou minéraux.

12) Les composés de formule (I) tels que définis à l'une quelconque des revendications 1 à 7 dans lesquels R₁ repré représente un radical : 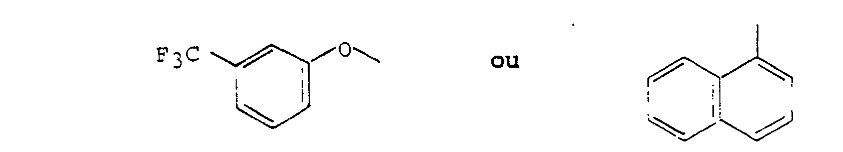 ainsi que leurs sels d'addition avec les acides organiques ou minéraux.

13) Les composés de formule (I) tels que définis à la revendication 1 dont les noms suivent :
- N-[(6-sec-butyl 2,4-dichloro phénoxy) acétyl] diamino-1,4 butane,
- 3,3'-[N-(6-sec-butyl 2,4-dichlorophénoxy) acétyl] 3,3'-diaminodipropylamine,
- N-(7-acétoxy coumarinyl) diamino-1,4 butane,
- N-[(6-sec-butyl 2,4-dichlorophénoxy) acétyl] NN'-spermidine,
- N-[(6-sec-butyl 2,4-dichlorophénoxy) acétyl] 4-guanidine amino butane,
- N-[(6-sec-butyl 2,4-dichlorophénoxy) acétyl] diamino 1,3-propane,
- N-[(6-sec-butyl 2,4-dichlorophénoxy) acétyl] diamino 1,5-pentane,
ainsi que leurs sels d'addition avec les acides organiques ou minéraux.

14) Procédé de préparation des composés de formule (1) tels que définis à l'une quelconque des revendications 1 à 13, caractérisé en ce que l'on soumet un acide de formule (II) : 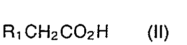 dans laquelle R₁ conserve sa signification précédente ou un dérivé fonctionnel de cet acide, à l'action d'une polyamine de formule (III) : 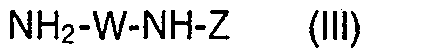 dans laquelle W et Z conservent la même signification que précédemment, pour obtenir le composé de formule (I) correspondant, que l'on soumet si désiré dans le cas où Z est un atome d'hydrogène à l'action d'un agent de fonctionnalisation de la fonction amine, puis soumet si désiré le composé de formule (I) obtenu à l'action d'un acide pour en former le sel.

15) A titre de produits industriels nouveaux les composés de formule (III) tels que définis à la revendication 14 dans lesquels W représente le radical -(CH₂)n₄NH(CH₂)n₅- dans lesquels n₄ et ns sont définis comme précédemment ainsi que ceux pour lesquels 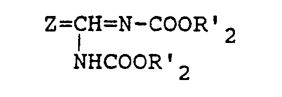 et W = (CH₂)ₙ avec n variant de 2 à 6.

16) Procédé de préparation des composés de formule (I) défini à la revendication 1 dans lesquels R₁ est un radical ArO-caractérisé en ce que l'on soumet un composé de formule (VI) : 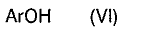 à l'action d'un composé de formule : 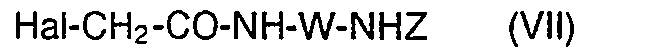 dans laquelle W et Z conservent leur signification précédente et Hal représente un atome d'halogène pour obtenir le composé de formule (I_{B}) correspondant : 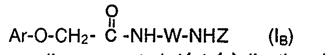 que l'on soumet si desire a l'action d'un aciae pour en former ie sei.

17) Les compositions fongicides renfermant comme principe actif au moins un composé de formule (I) tel que défini à la revendication 1, ainsi que leurs sels d'addition avec les acides organiques ou minéraux.

18) Les compositions fongicides renfermant comme principe actif au moins un composé de formule (I) tel que défini à l'une quelconque des revendications 2 à 12, ainsi que leurs sels d'addition avec les acides organiques ou minéraux.

19) Les compositions fongicides renfermant comme principe actif au moins un composé de formule (I) tel que défini à la revendication 13 ainsi que leurs sels d'addition avec les acides organiques ou minéraux. Revendications pour l'Etat contractant suivant: ES

1) Procédé pour préparer des composés de formule générale (1) : 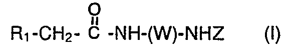 dans laquelle,
R représente :
soit un radical ArO- dans lequel Ar représente un radical aryle renfermant jusqu'à 14 atomes de carbone éventuellement substitué ou un radical hétéroaryle éventuellement substitué ou un radical hétérocyclique éventuellement substitué,
- soit un radical aryle ou polyaryle condensé ou non ou un radical hétérocyclique éventuellement substitué. W représente :
- soit un radical (CH₂)ₙ₁ dans lequel n₁ représente un nombre entier pouvant varier de 2 à 6,
- soit un radical (CH₂)n₂ NY(CH₂)n₃ dans lequel n₂ et n₃ identiques ou différents représentent un nombre entier pouvant varier de 2 à 6 et Y représente un atome d'hydrogène ou un radical alkyle renfermant jusqu'à 12 atomes de carbone ou un radical aryle renfermant jusqu'à 14 atomes de carbone éventuellement substitué ou un radical arylalkyle renfermant jusqu'à 18 atomes de carbone éventuellement substitué, ou un radical C0₂-alkyle dans lequel le radical alkyle renferme jusqu'à 12 atomes de carbone, ou un radical COR₂ dans lequel le radical R₂ représente un radical alkyle, alcényle ou alcynyle renfermant jusqu'à 12 atomes de carbone ou un radical aryle renfermant jusqu'à 14 atomes de carbone ou un radical arylalkyle renfermant jusqu'à 18 atomes de carbone éventuellement substitué ou un radical hétérocyclique éventuellement substitué et
Z représente :
- soit un atome d'hydrogène,
- soit un radical 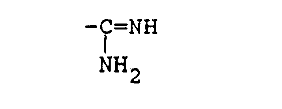
- soit un radical 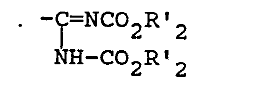 dans lequel R'₂ représente un radical alkyle renfermant jusqu'à 12 atomes de carbone, ou un radical arylalkyle renfermant jusqu'à 18 atomes de carbone,
- soit un radical C0₂-alkyle dans lequel le radical alkyle renferme jusqu'à 12 atomes de carbone,
- soit un radical C0₂-arylalkyle dans lequel le radical arylalkyle renferme jusqu'à 18 atomes de carbone,
- soit un radical COR"₂ dans lequel R"₂ peut prendre l'une des valeurs indiquées ci-dessus pour R₂,
- soit un radical -CH₂R₃ dans lequel R₃ représente un radical aryle éventuellement substitué, hétéroaryle éventuellement substitué, hétérocyclique éventuellement substitué ou peut prendre les valeurs indiquées ci-dessus pour R₁,
- soit un radical -COCH₂R₃ dans lequel R'₃ peut prendre l'une des valeurs indiquées ci-dessus pour R₃, à la condition que Z ne représente pas un atome d'hydrogène, si W représente un radical (CH₂)₃ et R₁ représente un radical 2,4-dichlorophénoxy ainsi que les sels d'addition avec les acides organiques ou minéraux des composés de formule (I),
caractérisé en ce que l'on soumet un acide de formule (II) : 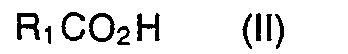 dans laquelle R, conserve sa signification précédente ou un dérivé fonctionnel de cet acide, à l'action d'une polyamine de formule (III) : 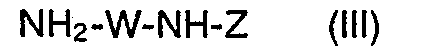 dans laquelle W et Z conservent la même signification que précédemment, pour obtenir le composé de formule (I) correspondant, que l'on soumet si désiré dans le cas où Z est un atome d'hydrogène à l'action d'un agent de fonctionnalisation de la fonction amine, puis soumet si désiré le composé de formule (I) obtenu à l'action d'un acide pour en former le sel.

2) Procédé selon la revendication 1, pour la préparation des produits de formule (I') : 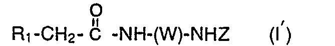 dans laquelle,
R, représente:
- soit un radical ArO- dans lequel Ar représente un radical aryle renfermant jusqu'à 14 atomes de carbone éventuellement substitué ou un radical hétéroaryle éventuellement substitué, ou un radical hétérocyclique éventuellement substitué,
- soit un radical aryle, ou polyaryle condensé ou non ou un radical hétérocyclique éventuellement substitué. W représente :
- soit un radical (CH₂)n₁ dans lequel n, représente un nombre entier pouvant varier de 3 à 6,
- soit un radical (CH₂)n₂ NY(CH₂)n₃ dans lequel n₂ et n₃ identiques ou différents représentent un nombre entier pouvant varier de 2 à 6 et Y représente un atome d'hydrogène ou un radical alkyle renfermant jusqu'à 12 atomes de carbone ou un radical aryle renfermant jusqu'à 14 atomes de carbone éventuellement substitué ou un radical arylalkyle renfermant jusqu'à 18 atomes de carbone éventuellement substitué, ou un radical C0₂ alkyle dans lequel le radical alkyle renferme jusqu'à 12 atomes de carbone, ou un radical COR₂ dans lequel le radical R₂ représente un radical alkyle, alcényle ou alcynyle renfermant jusqu'à 12 atomes de carbone ou un radical aryle renfermant jusqu'à 14 atomes de carbone ou un radical arylalkyle renfermant jusqu'à 18 atomes de carbone éventuellement substitué ou un radical hétérocyclique éventuellement substitué et
Z représente :
soit un atome d'hydrogène,
- soit un radical 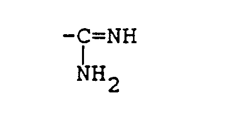
- soit un radical 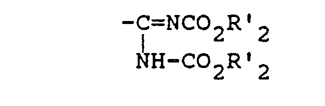 dans lequel R'₂ représente un radical alkyle renfermant jusqu'à 12 atomes de carbone, ou un radical arylalkyle renfermant jusqu'à 18 atomes de carbone,
- soit un radical C0₂ alkyle dans lequel le radical alkyle renferme jusqu'à 12 atomes de carbone,
- soit un radical C0₂ arylalkyle dans lequel le radical arylalkyle renferme jusqu'à 18 atomes de carbone,
- soit un radical -CH₂R₃ dans lequel R₃ représente un radical aryle éventuellement substitué, hétéroaryle éventuellement substitué, hétérocyclique éventuellement substitué ou peut prendre les valeurs indiquées ci-dessus pour Ri,
- soit un radical -COCH₂R₃ dans lequel R'₃ peut prendre l'une des valeurs indiquées ci-dessus pour R₃, à la condition que R₁ ne représente ni un radical 2,5-dihydroxyphényle, ni un radical 2,5-diméthoxyphényle et que Z ne représente pas un atome d'hydrogène, si W représente un radical (CH₂)₃ et R₁ représente un radical 2,4-dichlorophénoxy ainsi que les sels d'addition avec les acides organiques ou minéraux des composés de formule (I'), caractérisé en ce que l'on soumet un acide de formule (II) : 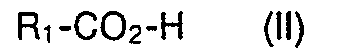 dans laquelle R₁ est défini comme ci-dessus à la condition qu'il ne représente ni un radical 2,5-dihydroxyphényle ni un radical 2,5-diméthoxyphényle, ou un dérivé fonctionnel de cet acide à l'action d'une polyamine de formule (III) : 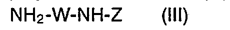 dans laquelle W et Z conservent la même signification que précédemment pour obtenir le composé de formule (I') correspondant que l'on soumet si désiré dans le cas où Z est un atome d'hydrogène à l'action d'un agent de fonctionnalisation de la fonction amine, puis soumet si désiré le composé de formule (I') obtenu à l'action d'un acide pour en former le sel étant entendu en outre que les produits de formule (II) et (III) sont choisis de telle façon que Z ne représente pas un atome d'hydrogène si W représente un radical (CH₂)₃ et R₁ représente un radical 2,4-dichlorophénoxy.

3) Procédé de préparation des composés de formule (I) défini à la revendication 1 dans lesquels Ri est un radical ArO- caractérisé en ce que l'on soumet un composé de formule (VI) :
ArOH (VI)
à l'action d'un composé de formule : 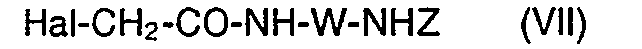 dans laquelle W et Z conservent leur signification précédente et Hal représente un atome d'halogène pour obtenir le composé de formule (I_{B}) correspondant : 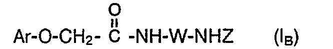 que l'on soumet si désiré à l'action d'un acide pour en former le sel.

4) Procédé selon la revendication 1, 2 ou 3, caractérisé en ce que l'on utilise au départ des produits de formule (II) ou (III) dans lesquelles les radicaux aryle, hétéroaryle, arylalkyle et hétérocyclique sont éventuellement substitués par un ou plusieurs substituants choisis dans le groupe constitué par les radicaux hydroxyle, les atomes d'halogène, les radicaux alkyle, alcényle et alcynyle renfermant jusqu'à 14 atomes de carbone, les radicaux CF₃, les radicaux OR"3 dans lequel R"₃ représente un radical alkyle renfermant jusqu'à 12 atomes de carbone ou un radical arylalkyle renfermant jusqu'à 18 atomes de carbone, les radicaux COalkyle, Si(alkyle)₃, SO₂alkyle dans lesquels le radical alkyle renferme jusqu'à 12 atomes de carbone, et les radicaux SO₂aryle dans lesquels le radical aryle renferme jusqu'à 14 atomes de carbone, les différents radicaux pouvant former entre eux avec les atomes auxquels ils sont liés des cycles 0-CH₂-0.

5) Procédé selon l'une quelconque des revendication 1 à 4, caractérisé en ce que l'on utilise au départ un produit de formule (III) dans laquelle W représente un radical (CH₂)₃-(CH₂)₄- ou (CH₂)₅-.

6) Procédé selon l'une quelconque des revendication 1 à 5, caractérisé en ce que l'on utilise au départ un produit de formule (III) dans laquelle W représente un radical (CH2)n4NH(CH2)ns dans lequel n₄ et ns identiques ou différents représentent les nombres 3, 4 ou 5.

7) Procédé selon l'une quelconque des revendication 1 à 6, caractérisé en ce que l'on utilise au départ un produit de formule (III) dans laquelle Z représente un atome d'hydrogène, ou un radical C0₂alkyle dans lequel le radical alkyle renferme jusqu'à 12 atomes de carbone.

8) Procédé selon l'une quelconque des revendication 1 à 7, caractérisé en ce que l'on utilise au départ un produit de formule (III) dans laquelle Z représente un radical 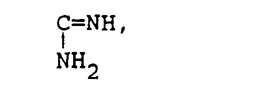 ou un radical 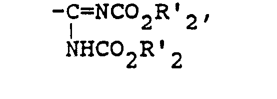 R'₂ gardant la même signification que dans la revendication 1.

9) Procédé selon l'une quelconque des revendication 1 à 8, caractérisé en ce que l'on utilise au départ un produit de formule (II) dans laquelle R₁ représente un radical ArO- dans lequel Ar représente un radical aryle éventuellement substitué.

10) Procédé selon la revendication 9, caractérisé en ce que Ar représente le radical :

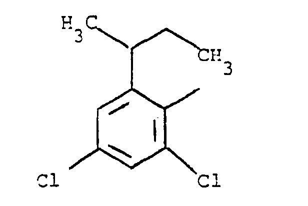

11) Procédé selon la revendication 9, caractérisé en ce que R, représente un radical :

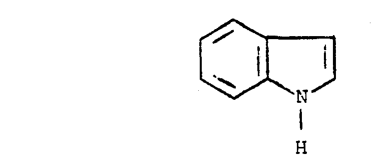

12) Procédé selon la revendication 9, caractérisé en ce que R₁ représente un radical :

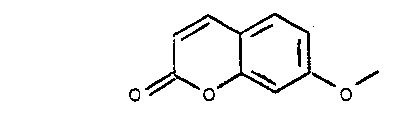

13) Procédé selon la revendication 9, caractérisé en ce que R₁ représente un radical :
